# EUROPEAN PATENT APPLICATION

(11) **EP 4 741 830 A1**
(43) Date of publication of application: **13.05.2026**
(21) Application number: 24838728.4
(22) Date of filing: 05.07.2024
(51) Int. Cl.: G01N 35/00

(54) **SAMPLE ANALYZER, PROTEIN ANALYSIS METHOD, AND PROTEIN ANALYSIS REAGENT**

(30) Priority: 07.07.2023 WO PCT/CN2023/106378
(71) Applicant: Shenzhen Mindray Bio-Medical Electronics Co., Ltd., Shenzhen, Guangdong 518057 (CN)
(72) Inventor: HUANG, Bin, Shenzhen, Guangdong 518057 (CN); WANG, Jin, Shenzhen, Guangdong 518057 (CN); JIANG, Ming, Shenzhen, Guangdong 518057 (CN)
(74) Representative: KIPA AB
(86) International application number: PCT/CN2024/103925
(87) International publication number: WO 2025/011475

(57) **Abstract**

The present application relates to a sample analyzer, a protein analysis method, and a protein analysis reagent. The protein analysis method comprises: preparing a first test solution by using a sample to be tested and a colorimetric reagent; testing the first test solution by using a colorimetric method, so as to acquire a colorimetric detection result for protein in said sample; on the basis of the colorimetric detection result, determining whether to perform a turbidimetric test; if a turbidimetric test is not performed, determining the concentration of the protein in said sample on the basis of the colorimetric detection result; and if a turbidimetric test is performed, preparing a second test solution by using said sample and a turbidimetric reagent, detecting the second test solution by using a turbidimetric method, so as to acquire a turbidimetric detection result for the protein in said sample, and determining the concentration of the protein in said sample on the basis of the turbidimetric detection result and the colorimetric detection result, optionally. The present application can integrate a colorimetric method with a turbidimetric method to measure protein in a sample, thereby enlarging the linear detection range of a single protein assay.

## Description

### TECHNICAL FIELD

The present disclosure relates to the field of in vitro diagnostics, and in particular to a sample analyzer, a protein analysis method and a protein analysis reagent.

### BACKGROUND

Currently, in an automated biochemical analyzer, there are mainly two methods, colorimetric method and turbidimetric method, which are used to measure protein in a sample, such as albumin in urine.

The basic principle of the colorimetric method is that: a dye (or coloring agent, chromogen) binds to protein, such as albumin, under specific conditions to produce a color change, thereby performing protein detection, for example, albumin detection. However, the colorimetric method has a low sensitivity and is generally used only to determine albumin content in serum, but usually not used to detect albumin in urine.

The basic principle of the turbidimetric method, such as immunoturbidimetric method, which is conventionally used to detect urinary albumin (urinary microalbumin) is that: an antibody binds to protein, such as albumin, to form turbidity for detection. However, the detection upper limit of the immunoturbidimetric method usually only covers a lower detection range, for example less than 1000 mg/L, with a hook effect. The concentration range of urinary albumin in a patient can reach a level of 30000 mg/L. Therefore, for a high-concentration urine sample, the immunoturbidimetric method is prone to a hook effect, resulting in misjudgment of results. In order to avoid the misjudgment caused by the hook effect, frequent dilution and retesting are required, resulting in a significant additional investment of resources, such as reagents.

### SUMMARY

In order to at least partially solve the technical problems described above, an object of the disclosure is to provide a sample analyzer, a protein analysis method and a protein analysis reagent, which are capable of combining two protein assay methods with different detection ranges to broaden the linear detection range of a single protein assay, for example, combining the low detection lower limit of the immunoturbidimetric method and the high detection upper limit of the colorimetric method to broaden the linear detection range for the protein assay.

In order to achieve the above object of the disclosure, a first aspect of the disclosure provides a sample analyzer, including:
a sample preparation apparatus configured to prepare a test solution to be tested from a sample and a detection reagent;
a detection device configured to detect the test solution to be tested, so as to obtain a detection result for protein in the test solution to be tested;
a controller configured to: control the sample preparation apparatus to prepare a first test solution by using a sample to be tested and a colorimetric reagent, where the colorimetric reagent includes at least a coloring agent, and control the detection device to detect the first test solution by using a colorimetric method, so as to acquire a colorimetric detection result for protein in the sample to be tested;
characterized in that the controller is further configured to:
   determine, on the basis of the colorimetric detection result, whether to perform a turbidimetric test, if no, then determine a concentration of the protein in the sample to be tested on the basis of the colorimetric detection result, and if yes, then control the sample preparation apparatus to prepare a second test solution by using the sample to be tested and a turbidimetric reagent, and control the detection device to detect the second test solution by using a turbidimetric method, so as to acquire a turbidimetric detection result for the protein in the sample to be tested, and determine a concentration of the protein in the sample to be tested on the basis of the turbidimetric detection result and optionally the colorimetric detection result; or,
   control the sample preparation apparatus to prepare a second test solution by using the sample to be tested and a turbidimetric reagent, and control the detection device to detect the second test solution by using a turbidimetric method, so as to acquire a turbidimetric detection result for the protein in the sample to be tested, and select, on the basis of the colorimetric detection result, at least one of the colorimetric detection result and the turbidimetric detection result to determine a concentration of the protein in the sample to be tested.

A second aspect of the disclosure provides another sample analyzer, including:
a sample preparation apparatus configured to prepare a test solution to be tested from a sample and a detection reagent;
a detection device configured to detect the test solution to be tested, so as to obtain a detection result for protein in the test solution to be tested;
a controller configured to: control the sample preparation apparatus to prepare a first test solution by using a sample to be tested and a turbidimetric reagent; and control the detection device to detect the first test solution by using a turbidimetric method, so as to acquire a turbidimetric detection result for protein in the sample to be tested;
characterized in that the controller is further configured to:
   determine, on the basis of the turbidimetric detection result, whether to perform a colorimetric test, if no, then determine a concentration of the protein in the sample to be tested on the basis of the colorimetric detection result, and if yes, then control the sample preparation apparatus to prepare a second test solution by using the sample to be tested and a colorimetric reagent, and control the detection device to detect the second test solution by using a colorimetric method, so as to acquire a colorimetric detection result for the protein in the sample to be tested, and determine a concentration of the protein in the sample to be tested on the basis of the colorimetric detection result and optionally the turbidimetric detection result, where the colorimetric reagent includes at least a coloring agent; or,
   control the sample preparation apparatus to prepare a second test solution by using the sample to be tested and a colorimetric reagent, control the detection device to detect the second test solution by using a colorimetric method, so as to acquire a colorimetric detection result for the protein in the sample to be tested, where the colorimetric reagent includes at least a coloring agent, and select, on the basis of the turbidimetric detection result, at least one of the colorimetric detection result and the turbidimetric detection result to determine a concentration of the protein in the sample to be tested.

A third aspect of the disclosure provides yet another sample analyzer, including:
a sample preparation apparatus configured to prepare a test solution to be tested from a sample;
a detection device configured to detect the test solution to be tested, so as to obtain a detection result for protein in the test solution to be tested;
a controller configured to: on the basis of a first detection method, control the sample preparation apparatus to prepare a first test solution by using a sample to be tested and a first detection reagent and control the detection device to detect the first test solution, so as to acquire a first detection result for protein in the sample to be tested;
characterized in that the controller is further configured to:
   determine, on the basis of the first detection result, whether to perform a test on the basis of a second detection method, if no, then determine a concentration of the protein in the sample to be tested on the basis of the first detection result, and if yes, then on the basis of the second detection method, control the sample preparation apparatus to prepare a second test solution by using the sample to be tested, and control the detection device to detect the second test solution, so as to acquire a second detection result for the protein in the sample to be tested, and determine a concentration of the protein in the sample to be tested on the basis of the second detection result and the first detection result; or,
   on the basis of the second detection method, control the sample preparation apparatus to prepare a second test solution by using the sample to be tested, and control the detection device to detect the second test solution, so as to acquire a second detection result for the protein in the sample to be tested, and select, on the basis of the first detection result, at least one of the first detection result and the second detection result to determine a concentration of the protein in the sample to be tested.

A fourth aspect of the disclosure provides still another sample analyzer, including:
a sample preparation apparatus configured to prepare a test solution to be tested from a sample;
a detection device configured to test the test solution to be tested, so as to obtain a detection result for protein in the test solution to be tested;
a controller configured to: on the basis of a first detection method, control the sample preparation apparatus to prepare a first test solution by using a sample to be tested and a first detection reagent, and control the detection device to detect the first test solution, so as to acquire a first detection result for protein in the sample to be tested;
characterized in that the controller is further configured to:
   determine, on the basis of the first detection result, whether to perform a test on the basis of a second detection method, if no, then determine a concentration of the protein in the sample to be tested on the basis of the first detection result, and if yes, then on the basis of the second detection method, control the sample preparation apparatus to prepare a second test solution by using the sample to be tested, and control the detection device to detect the second test solution, so as to acquire a second detection result for the protein in the sample to be tested, and determine a concentration of the protein in the sample to be tested on the basis of the second detection result and optionally the first detection result; or,
   on the basis of the second detection method, control the sample preparation apparatus to prepare a second test solution by using the sample to be tested, and control the detection device to detect the second test solution, so as to acquire a second detection result for the protein in the sample to be tested, and select, on the basis of the first detection result, at least one of the first detection result and the second detection result to determine a concentration of the protein in the sample to be tested.

A fifth aspect of the disclosure provides a protein analysis method for a sample analyzer, where the sample analyzer includes a sample preparation apparatus, a detection device, and a controller, and the protein analysis method includes: preparing a first test solution by the sample preparation apparatus by using a sample to be tested and a colorimetric reagent, where the colorimetric reagent includes at least a coloring agent; and detecting the first test solution by the detection device by using a colorimetric method, so as to acquire a colorimetric detection result for protein in the sample to be tested;
characterized in that the protein analysis method further includes performing the following steps by the controller:
determining, on the basis of the colorimetric detection result, whether to perform a turbidimetric test, if no, then determining a concentration of the protein in the sample to be tested on the basis of the colorimetric detection result, and if yes, then controlling the sample preparation apparatus to prepare a second test solution by using the sample to be tested and a turbidimetric reagent, and controlling the detection device to detect the second test solution by using a turbidimetric method, so as to acquire a turbidimetric detection result for the protein in the sample to be tested, and determining a concentration of the protein in the sample to be tested on the basis of the turbidimetric detection result and optionally the colorimetric detection result; or,
controlling the sample preparation apparatus to prepare a second test solution by using the sample to be tested and a turbidimetric reagent, and controlling the detection device to detect the second test solution by using a turbidimetric method, so as to acquire a turbidimetric detection result for the protein in the sample to be tested, and selecting, on the basis of the colorimetric detection result, at least one of the colorimetric detection result and the turbidimetric detection result to determine a concentration of the protein in the sample to be tested.

A sixth aspect of the disclosure provides another protein analysis method for a sample analyzer, where the sample analyzer includes a sample preparation apparatus, a detection device, and a controller, and the protein analysis method includes: preparing a first test solution by the sample preparation apparatus by using a sample to be tested and a turbidimetric reagent; and detecting the first test solution by the detection device by using a turbidimetric method, so as to acquire a turbidimetric detection result for the protein in the sample to be tested;
characterized in that the protein analysis method further includes performing the following steps by the controller:
determining, on the basis of the turbidimetric detection result, whether to perform a colorimetric test, if no, then determining a concentration of the protein in the sample to be tested on the basis of the colorimetric detection result, and if yes, then controlling the sample preparation apparatus to prepare a second test solution by using the sample to be tested and a colorimetric reagent, and controlling the detection device to detect the second test solution by using a colorimetric method, so as to acquire a colorimetric detection result for the protein in the sample to be tested, and determining a concentration of the protein in the sample to be tested on the basis of the colorimetric detection result and optionally the turbidimetric detection result, where the colorimetric reagent includes at least a coloring agent; or,
controlling the sample preparation apparatus to prepare a second test solution by using the sample to be tested and a colorimetric reagent, controlling the detection device to detect the second test solution by using a colorimetric method, so as to acquire a colorimetric detection result for the protein in the sample to be tested, where the colorimetric reagent includes at least a coloring agent, and selecting, on the basis of the turbidimetric detection result, at least one of the colorimetric detection result and the turbidimetric detection result to determine a concentration of the protein in the sample to be tested.

A seventh aspect of the disclosure provides a protein analysis reagent, including:
a colorimetric reagent including a coloring agent; and
a turbidimetric reagent including an antibody reagent or a protein precipitation reagent.

### BRIEF DESCRIPTION OF THE DRAWINGS

FIG. 1 is a schematic block diagram of a sample analyzer provided according to an embodiment of the disclosure.
FIG. 2 is a schematic diagram showing a structure of an example of the sample analyzer of FIG. 1.
FIG. 3 is a schematic flowchart of a protein analysis method provided according to a first example of the disclosure.
FIG. 4 is a schematic flowchart of a protein analysis method provided according to a second example of the disclosure.
FIG. 5 is a schematic flowchart of a protein analysis method provided according to a third example of the disclosure.
FIG. 6 is a schematic flowchart of a protein analysis method provided according to a fourth example of the disclosure.
FIG. 7 is a result curve graph of the detection of albumin in urine by using a colorimetric method according to the prior art.
FIG. 8 is a result curve graph of the detection of albumin in urine by using a turbidimetric method according to the prior art.
FIG. 9 is a reaction process for detecting albumin in a urine sample.
FIG. 10 is a result curve graph of the detection of albumin in urine according to an embodiment of the disclosure.
FIG. 11 is a result curve graph of the detection of total protein in urine by using a colorimetric method according to the prior art.
FIG. 12 is a result curve graph of the detection of total protein in urine by using a turbidimetric method according to the prior art.
FIG. 13 is a reaction process for detecting total protein in a urine sample .
FIG. 14 is a result curve graph of the detection of total protein in urine according to an embodiment of the disclosure.
FIG. 15 is a target absorption curve and an interference absorption curve according to an embodiment of the disclosure.

### DESCRIPTION OF THE PREFERRED EMBODIMENTS

The technical solutions of the embodiments of the disclosure will be described below clearly and comprehensively with reference to the accompanying drawings of the embodiments of the disclosure. Apparently, the embodiments described are merely some of, rather than all of, the embodiments of the disclosure. Based on the embodiments of the disclosure, all the other embodiments that would have been obtained by those of ordinary skill in the art without any creative efforts shall fall within the scope of protection of the disclosure.

The turbidimetric method is a method known in the art for determining the amount or degree of turbidity in a solution based on measuring the effect of turbidity on the transmission and scattering of light. Turbidity in a liquid is caused by the presence of dispersed suspended particles. The turbidimetric method includes transmission turbidimetry (turbidimetry) and scatter turbidimetry (nephelometry). In transmission turbidimetry, the intensity of light transmitted through a sample is measured; whereas in scatter turbidimetry, the intensity of the scattered light is measured. The principle of detecting albumin in a sample on the basis of the immunoturbidimetric method is as follows: albumin in the sample binds to antibodies to form insoluble substance, e.g., in the environment of a turbidity-enhancing agent, resulting in turbidity; the instrument reads the absorbance change information and compares the information with a standard curve to obtain the concentration result of albumin. This method has a high sensitivity (i.e., a low detection lower limit) and is capable of accurately detecting low-concentration albumin, so this method is the mainstream method for detecting albumin in urine. However, when the concentration of albumin in urine is high, a hook effect occurs, making it impossible to provide accurate results.

The colorimetric method (colorimetry) is a method for qualitative and quantitative analysis of analytes by measuring the absorbance of light in a solution at a specific wavelength or within a certain wavelength range. The principle of detecting albumin in a sample on the basis of the colorimetric method is as follows: albumin binds to a coloring agent to produce a color change, and the instrument reads absorbance information at a specific wavelength, and then compares the information with a standard curve to obtain the concentration result of albumin. This method has a low sensitivity but a high detection upper limit, allowing accurate detection of albumin at relatively high concentrations.

Based on this, the present disclosure proposes a technical solution for improving the protein detection performance of a sample analyzer, in which two reagents/methods with different characteristics for detecting protein in a biological sample are combined, so that the sample analyzer has a wider protein detection range.

The embodiments of the disclosure are suitable for detecting a protein in a biological sample, especially albumin or total protein. The biological sample referred to in the embodiments of the disclosure is urine or serum or cerebrospinal fluid. It is particularly preferred that the embodiments of the disclosure are suitable for detecting a protein in urine, especially albumin or total protein in urine.

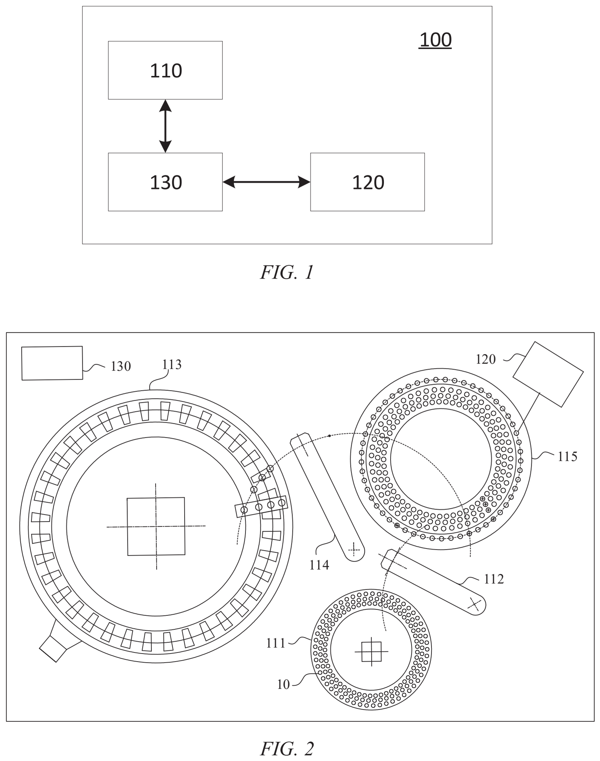

FIG. 1 is a schematic block diagram of a sample analyzer according to an embodiment of the disclosure. As shown in FIG. 1, the sample analyzer 100 includes a sample preparation apparatus 110, a detection device 120, and a controller 130.

The sample preparation apparatus 110 is configured to prepare a test solution to be tested from a sample. For example, the sample preparation apparatus 110 is configured to mix a sample to be tested, such as a urine to be tested, with a corresponding detection reagent, such as a coloring agent or an antibody reagent, to prepare a test solution to be tested.

The detection device 120 is configured to detect the test solution to be tested, so as to obtain a detection result of protein in the test solution to be tested.

The controller 130 is configured to: on the basis of a first detection method, control the sample preparation apparatus 110 to prepare a first test solution by using a sample to be tested and a first detection reagent, and control the detection device 120 to detect the first test solution, so as to acquire a first detection result for protein in the sample to be tested.

According to a first embodiment of the disclosure, the controller 130 is further configured to:
determine, on the basis of the first detection result, whether to perform a test on the basis of a second detection method;
if no, that is, if it is determined not to perform the test on the basis of the second detection method, then determine a concentration of the protein in the sample to be tested on the basis of the first detection result;
if yes, that is, if it is determined to perform the test on the basis of the second detection method, then on the basis of the second detection method, control the sample preparation apparatus 110 to prepare a second test solution by using the sample to be tested, and control the detection device 120 to detect the second test solution, so as to acquire a second detection result for the protein in the sample to be tested, and determine a concentration of the protein in the sample to be tested on the basis of the second detection result and the first detection result.

That is, in the first embodiment according to the disclosure, first, on the basis of the first detection method, the first test solution is prepared by using the sample to be tested and is detected, so as to acquire the first detection result for the protein in the sample to be tested; then, it is determined, on the basis of the first detection result, whether a protein detection on the basis of the second detection method different from the first detection method is required to be performed for the sample to be tested; if the protein detection on the basis of the second detection method is not required to be performed for the sample to be tested, then the concentration of the protein in the sample to be tested is determined on the basis of the first detection result, in particular only on the basis of the first detection result; if the protein detection on the basis of the second detection method is required to be performed for the sample to be tested, then on the basis of the second detection method, the second test solution is prepared by using the sample to be tested and is detected, so as to acquire the second detection result for the protein in the sample to be tested, and the concentration of the protein in the sample to be tested is determined on the basis of the second detection result and the first detection result.

With this first embodiment, not only can the linear detection range of protein detection be broadened, but also can avoid retesting samples with high protein concentrations, thus reducing costs.

Alternatively, according to a second embodiment of the disclosure, the controller 130 is further configured to:
on the basis of a second detection method, control the sample preparation apparatus 110 to prepare a second test solution by using the sample to be tested and control the detection device 120 to detect the second test solution, so as to acquire a second detection result for the protein in the sample to be tested; and
select, on the basis of the first detection result, at least one of the first detection result and the second detection result to determine a concentration of the protein in the sample to be tested.

That is, in the second embodiment according to the disclosure, first, on the basis of the first detection method, the first test solution is prepared by using the sample to be tested and is detected, so as to acquire the first detection result for the protein in the sample to be tested, and then on the basis of the second detection method, the second test solution is prepared by using the sample to be tested and is detected, so as to acquire the second detection result for the protein in the sample to be tested; then, on the basis of the first detection result, at least one of the first detection result and the second detection result is selected to determine the concentration of the protein in the sample to be tested.

With this second embodiment, the linear detection range for protein detection can be broadened.

Alternatively, according to a third embodiment of the disclosure, the controller 130 is further configured to: determine, on the basis of the first detection result, whether to perform a test on the basis of a second detection method, if no, then determine a concentration of the protein in the sample to be tested on the basis of the first detection result, and if yes, then on the basis of the second detection method, control the sample preparation apparatus to prepare a second test solution by using the sample to be tested and a second detection reagent, and control the detection device to detect the second test solution, so as to acquire a second detection result for the protein in the sample to be tested, and determine a concentration of the protein in the sample to be tested on the basis of the second detection result and optionally the first detection result.

That is, in the third embodiment according to the disclosure, first, on the basis of the first detection method, the first test solution is prepared by using a sample to be tested and is detected, so as to acquire the first detection result for the protein in the sample to be tested; then, it is determined, on the basis of the first detection result, whether a protein detection on the basis of the second detection method different from the first detection method is required to be performed for the sample to be tested; if the protein detection on the basis of the second detection method is not required to be performed for the sample to be tested, then the concentration of protein in the sample to be tested is determined on the basis of the first detection result, in particular only on the basis of the first detection result; if the protein detection on the basis of the second detection method different is required to be performed for the sample to be tested, then on the basis of the second detection method, a second test solution is prepared by using the sample to be tested and a second detection reagent and is detected, so as to acquire the second detection result for the protein in the sample to be tested, and the concentration of the protein in the sample to be tested is determined on the basis of the second detection result and optionally the first detection result, for example, the concentration of the protein in the sample to be tested is calculated only on the basis of second detection result or on the basis of both the second detection result and the first detection result.

Alternatively, according to a fourth embodiment of the disclosure, the controller 130 is further configured to:
on the basis of a second detection method, control the sample preparation apparatus 110 to prepare a second test solution by using a sample to be tested and a second detection reagent and control the detection device 120 to detect the second test solution, so as to acquire a second detection result for the protein in the sample to be tested; and select, on the basis of the first detection result, at least one of the first detection result and the second detection result to determine a concentration of the protein in the sample to be tested.

That is, in the fourth embodiment according to the disclosure, first, on the basis of the first detection method, the first test solution is prepared by using the sample to be tested and is detected, so as to acquire the first detection result for the protein in the sample to be tested, and on the basis of the second detection method, the second test solution is prepared by using the sample to be tested and a second detection reagent and is detected, so as to acquire the second detection result for the protein in the sample to be tested; then, on the basis of the first detection result, at least one of the first detection result and the second detection result is selected to determine the concentration of the protein in the sample to be tested.

In embodiments of the disclosure, the first detection method and the second detection method for measuring a protein in a biological sample have different linear detection ranges. For example, the detection upper limit Max1 for protein concentration of the first detection method is greater than the detection upper limit Max2 for protein concentration of the second detection method, and the detection lower limit Min1 for protein concentration of the first detection method is greater than the detection lower limit Min2 for protein concentration of the second detection method, thus for example, the linear detection range [Min2, Max1] obtained by combining the first detection method and the second detection method is wider than the linear detection range [Min1, Max1] of the first detection method and the linear detection range [Min2, Max2] of the second detection method.

In embodiments of the disclosure, the first detection result may be a protein concentration or may be an intermediate value related to the protein concentration obtained during detection on the basis of the first detection method. Similarly, the second detection result may be a protein concentration or may be an intermediate value related to the protein concentration obtained during detection on the basis of the second detection method.

In embodiments of the disclosure, the first detection method and the second detection method differ from each other and differ in at least one of the following aspects:
a calibration curve corresponding to the first detection method and a calibration curve corresponding to the second detection method are different;
the first detection method and the second detection method are based on different methodologies;
the first detection reagent used in the first detection method and the second detection reagent used in the second detection method belong to different categories;
the first detection reagent used in the first detection method and the second detection reagent used in the second detection method belong to a same category, , but the first detection reagent used in the first testing method and the second detection reagent used in the second testing method differ in formulation, especially in concentration;
a detector used for optical signal measurement of the first test solution in the first detection method and a detector used for optical signal measurement of the second test solution in the second detection method belong to different categories or have different linear detection ranges; and
a detection wavelength used for optical signal measurement of the first test solution in the first detection method is different from a detection wavelength used for optical signal measurement of the second test solution in the second detection method.

As some implementations, a calibration curve corresponding to the first detection method and a calibration curve corresponding to the second detection method are different. Accordingly, the controller is further configured to: obtain the first detection result from a first calibration curve and first optical signals obtained by detecting the first test solution; obtain the second detection result from a second calibration curve and second optical signals obtained by detecting the second test solution; where the first calibration curve is different from the second calibration curve, preferably a linear range of the first calibration curve is different from that of the second calibration curve, and the linear range of the first calibration curve and the linear range of the second calibration curve partially overlap with each other.

As some other implementations, the first detection method and the second detection method are based on different methodologies or different detection principles.

In some embodiments, the first detection method and the second detection method are based on one of a colorimetric method and a turbidimetric method, respectively. For example, the first detection method is based on a colorimetric method and the second detection method is based on a turbidimetric method, such as an antibody reagent-based turbidimetric method or a protein precipitation reagent-based turbidimetric method, or the first detection method is based on a turbidimetric method, such as an antibody reagent-based turbidimetric method or a protein precipitation reagent-based turbidimetric method, and the second detection method is based on a colorimetric method. As some alternative or additional implementations, the first detection reagent used in the first detection method and the second detection reagent used in the second detection method belong to different categories.

In some embodiments, the first detection method is based on a colorimetric method and the first detection reagent is a colorimetric reagent, the second detection method is based on a turbidimetric method and the second detection reagent is a turbidimetric reagent. In some other embodiments, the first detection method is based on a turbidimetric method and the first detection reagent is a turbidimetric reagent, the second detection method is based on a colorimetric method and the second detection reagent is a colorimetric reagent. In some yet other embodiments, that first detection method and the second detection method are both based on the colorimetric method, and the colorimetric reagent of the first detection method and the colorimetric reagent of the second detection method belong to different categories. In some still other embodiments, the first detection method and the second detection method are both turbidimetric method, and the turbidimetric reagent of the first detection method and the turbidimetric reagent of the second detection method belong to different categories.

As some other implementations, the first detection reagent used in the first detection method and the second detection reagent used in the second detection method belong to a same category, but the first detection reagent used in the first detection method and the second detection reagent used in the second detection method differ in formulation, especially in concentration.

For example, both the first detection reagent used in the first detection method and the second detection reagent used in the second detection method include bromocresol green, but the concentration of bromocresol green in the first detection reagent is different from the concentration of bromocresol green in the second detection reagent. As another example,

As some other alternative or additional implementations, a detector used for optical signal measurement of the first test solution in the first detection method and a detector used for optical signal measurement of the second test solution in the second detection method belong to different categories or have different linear detection ranges.

In some embodiments, for example when the first detection method is based on a colorimetric method and the second detection method is based on a turbidimetric method, the detection device includes a transmitted light detector and a scattered light detector, the transmitted light detector being used for optical signal measurement of the first test solution, and the scattered light detector being used for optical signal measurement of the second test solution.

In some other embodiments, for example when the first detection method and the second detection method are both based on the colorimetric method, the detection device includes a first transmitted light detector and a second transmitted light detector, the first transmitted light detector being used for optical signal measurement of the first test solution, and the second transmitted light detector being used for optical signal measurement of the second test solution. In this case, the linear detection range of the first transmitted light detector is smaller than the linear detection range of the second transmitted light detector.

In some embodiment, the different linear detection ranges of the detectors may be caused by a circuit structure, algorithm, etc.

As some yet other implementations, a detection wavelength used for optical signal measurement of the first test solution in the first detection method is different from a detection wavelength used for optical signal measurement of the second test solution in the second detection method.

For example, in the first detection method, the detection device measures the absorbance change of the first test solution at a first detection wavelength, while in the second detection method, the detection device measures the absorbance change of the second test solution at a second detection wavelength different from the first detection wavelength.

FIG. 2 is a schematic diagram showing a structure of the sample analyzer 100 of FIG. 1, which is configured as a biochemical analyzer.

As shown in FIG. 2, the sample preparation apparatus 110 of the sample analyzer 100 includes a sample holding component 111, a sample dispensing component 112, a reagent holding component 113, a reagent dispensing component 114 and a reaction component 115.

The sample holding component 111 is configured to hold containers each containing a biological sample of a subject, such as urine. For example, the sample holding component 11 may be configured as a sample disc, the sample disc includes a plurality of sample positions capable of holding containers 10, and the sample disc can be rotated to dispatch each container containing the biological sample to a corresponding position, for example, to a position in which the sample dispensing component 112 aspirates the biological sample.

The sample dispensing component 112 is configured to aspirate the biological sample of the subject from the sample holding component 111 and discharge the biological sample into a reaction cup to be added with sample. For example, the sample dispensing component 112 may include a sample needle, which can perform a two-dimensional or three-dimensional movement in space by using a two-dimensional or three-dimensional driving mechanism, so that the sample needle can move to the position in which the biological sample is to be aspirated, move to the reaction cup to be added with sample, and discharge the aspirated biological sample into said reaction cup.

The reagent holding component 113 is configured to hold detection reagents, such as a coloring agent for the colorimetric method and an antibody reagent or a protein precipitation reagent for the turbidimetric method. For example, the reagent holding component 113 may be configured as a reagent disk having a circular disc structure and provided with a plurality of positions for holding reagent containers. The reagent holding component 113 can rotate and drive the reagent containers held therein to rotate to a specific position, for example, a position that allows the reagent dispensing component 114 to aspirate a reagent. There may be one or more reagent holding components 114.

The reagent dispensing component 114 is configured to aspirate a detection reagent from the reagent holding component 113 and discharge the detection reagent into a reaction cup to be added with reagent. For example, the reagent dispensing component 114 may include a reagent needle, which can perform a two-dimensional or three-dimensional movement in space by using a two-dimensional or three-dimensional driving mechanism, so that the reagent needle can move to the position in which the reagent is to be aspirated, move to the reaction cup to be added with reagent, and discharge the aspirated reagent into said reaction cup.

The reaction component 115 is configured to place a reaction cup in order to incubate a test solution from reaction of the biological sample of the subject with the detection reagent in said reaction cup. For example, the reaction component 115 may be configured as a reaction disc having a circular disc structure and provided with one or more placement positions each for placing a reaction cup. The reaction disc can rotate and drive each reaction cup in each placement position to move, so as to dispatch said reaction cup in the reaction disc and incubate the test solution in said reaction cup.

The detection device 120 of the sample analyzer 100 has an optical detector and is configured to determine a protein concentration in a biological sample of a subject. For example, the detection device 120 is arranged outside the reaction component 115, where the reaction component 115 rotates to drive a reaction cup containing a test solution to move to the detection device 120 for detection.

The controller 130 may include a processor and a computer-readable storage medium with computer-readable instructions stored thereon, wherein the computer-readable instructions, when executed by the processor, cause the processor to perform various steps of protein detection.

In some embodiments, the processor may include, but is not limited to, a central processing unit (CPU), a micro controller unit (MCU), a field-programmable gate array (FPGA), a digital signal processor (DSP) and other devices for interpreting computer instructions and processing data in computer software.

In some embodiments, the controller 130 further configured to determine, on the basis of the first detection result, whether to perform a test on the basis of a second detection method, may include: the controller 130 further configured to:
determine that the test on the basis of the second detection method is not required to be performed, if the first detection result is in a first predetermined range; and
determine that the test on the basis of the second detection method is required to be performed, if the first detection result is in a second predetermined range.

As some implementations, when a protein concentration detection upper limit of the first detection method is greater than a protein concentration detection upper limit of the second detection method, and optionally a protein concentration detection lower limit of the first detection method is greater than a protein concentration detection lower limit of the second detection method, the controller 130 further configured to determine, on the basis of the first detection result, whether to perform a test on the basis of a second detection method, may include: the controller 130 further configured to:
determine that the test on the basis of the second detection method is not required to be performed perform, if the first detection result is greater than a predetermined threshold value; and
determine that the test on the basis of the second detection method is required to be performed, if the first detection result is not greater than the predetermined threshold value.

The predetermined threshold value here may be determined empirically or may be determined according to the protein concentration detection upper limit of the second detection method and the protein concentration detection lower limit of the first detection method, for example, may be selected to be greater than the protein concentration detection lower limit of the first detection method and less than the protein concentration detection upper limit of the second detection method.

As some other implementations, when a protein concentration detection upper limit of the first detection method is less than a protein concentration detection upper limit of the second detection method, and optionally a protein concentration detection lower limit of the first detection method is less than a protein concentration detection lower limit of the second detection method, the controller 130 further configured to determine, on the basis of the first detection result, whether to perform a test on the basis of a second detection method, may include: the controller 130 further configured to:
determine that the test on the basis of the second detection method is not required to be performed, if the first detection result is less than a predetermined threshold value; and
determine that the test on the basis of the second detection method is required to be performed, if the first detection result is not less than the predetermined threshold value.

The predetermined threshold value here may be determined empirically or may be determined according to the protein concentration detection upper limit of the first detection method and the protein concentration detection lower limit of the second detection method, for example, may be selected to be greater than the protein concentration detection lower limit of the second detection method and less than the protein concentration detection upper limit of the first detection method.

According to some examples of the first embodiment of the disclosure, the first detection method is based on a colorimetric method and the second detection method is based on a turbidimetric method. Accordingly, the controller 130 may be configured to:
control the sample preparation apparatus 110 to prepare a first test solution by using a sample to be tested and a colorimetric reagent, where the colorimetric reagent includes at least a coloring agent, and control the detection device 120 to detect the first test solution by using a colorimetric method, so as to acquire a colorimetric detection result for protein in the sample to be tested;
determine whether to perform a turbidimetric test on the basis of the colorimetric detection result;
if no, then determine a concentration of the protein in the sample to be tested on the basis of the colorimetric detection result (e.g., determine the concentration of the protein in the sample to be tested only on the basis of the colorimetric detection result), and
if yes, then control the sample preparation apparatus 110 to prepare a second test solution by using the sample to be tested and a turbidimetric reagent, and control the detection device 120 to detect the second test solution by using a turbidimetric method, so as to acquire a turbidimetric detection result for the protein in the sample to be tested, and determine a concentration of the protein in the sample to be tested on the basis of the turbidimetric detection result and optionally the colorimetric detection result, (e.g., determine the concentration of the protein in the sample to be tested only on the basis of the turbidimetric detection result, or determine the concentration of the protein in the sample to be tested on the basis of both the turbidimetric detection result and the colorimetric detection result).

Further, the controller 130 configured to determine, on the basis of the colorimetric detection result, whether to perform a turbidimetric test, may include: the controller 130 configured to:
determine that the turbidimetric test is not required to be performed, if the colorimetric detection result is in a first predetermined range; and
determine that the turbidimetric test is required to be performed, if the colorimetric detection result is in a second predetermined range that is different from the first predetermined range.

In some embodiments, the second detection method is based on an immunoturbidimetric method, i.e., the turbidimetric reagent includes at least an antibody reagent. In this case, the sample analyzer is especially suitable for detecting albumin in a biological sample, preferably in urine. Accordingly, the controller 130 configured to determine, on the basis of the colorimetric detection result, whether to perform a turbidimetric test, may include: the controller 130 configured to:
determine that the turbidimetric test is not required to be performed if the colorimetric detection result is greater than a predetermined threshold value; and
determine that the turbidimetric test is required to be performed, if the colorimetric detection result is not greater than the predetermined threshold value.

Here, the sample analyzer is especially suitable for detecting albumin or microalbumin in urine. In this case, the albumin concentration detection upper limit of the colorimetric method is greater than the albumin concentration detection upper limit of the immunoturbidimetric method, and the albumin concentration detection lower limit of the colorimetric method is greater than the albumin concentration detection lower limit of the immunoturbidimetric method.

The predetermined threshold value here may be determined empirically or may be determined according to the albumin concentration detection upper limit of the immunoturbidimetric method and the albumin concentration detection lower limit of the colorimetric method, for example, may be selected to be substantially greater than the albumin concentration detection lower limit of the colorimetric method and substantially less than the albumin concentration detection upper limit of the turbidimetric method.

In a specific example, the reaction of the coloring agent and albumin (ALB) can lead to a change in the absorption spectrum of albumin, i.e. an increase/decrease in absorbance at certain wavelengths, that is, the absorbance at these wavelengths changes as the concentration of ALB changes. Thus, a certain absorbance may be set as the predetermined threshold value; it is also possible, taking into account the performance of the coloring agent, to correct the absorbance (such as multiplied by a coefficient of variation) to obtain the corrected absorbance as the predetermined threshold value; it is also possible to convert the absorbance to a sample concentration and set a certain sample concentration as the predetermined threshold value; it is also possible to determine the predetermined threshold value from calibrated data at the time of calibration, etc.

Alternatively, in some other embodiments, the second detection method is based on a turbidimetric method on the basis of a protein precipitation reagent, i.e., the turbidimetric reagent includes at least a protein precipitation reagent. In this case, the sample analyzer is especially suitable for detecting total protein in a biological sample, preferably in urine. Accordingly, the controller 130 configured to determine, on the basis of the colorimetric detection result, whether to perform a turbidimetric test, may include: the controller 130 configured to:
determine that the turbidimetric test is not required to be performed, if the colorimetric detection result is less than a predetermined threshold value; and
determine that the turbidimetric test is required to be performed, if the colorimetric detection result is not less than the predetermined threshold value.

Here, the sample analyzer is especially suitable for detecting total protein in urine. In this case, the total protein concentration detection upper limit of the colorimetric method is less than the total protein concentration detection upper limit of the turbidimetric method on the basis of the protein precipitation reagent, and the total protein concentration detection lower limit of the colorimetric method is less than the total protein concentration detection lower limit of the turbidimetric method on the basis of the protein precipitation reagent.

The predetermined threshold value here may be determined empirically or may be determined according to the total protein concentration detection upper limit of the colorimetric method and the total protein concentration detection lower limit of the turbidimetric method on the basis of the protein precipitation reagent, for example, may be selected to be substantially greater than the total protein concentration detection lower limit of the turbidimetric method on the basis of the protein precipitation reagent and substantially less than the total protein concentration detection upper limit of the colorimetric method.

In some embodiment, the predetermined threshold value may be determined based on light intensity signals or absorbance signals during reaction of the sample to be tested with the colorimetric reagent or based on a reaction curve of the sample to be tested with the colorimetric reagent. For example, the predetermined threshold value may be determined based on a protein concentration converted from the absorbance signals through a calibration curve, or may be determined based on a slope of a certain curve segment of the reaction curve.

In some other embodiments, the predetermined threshold value may also be determined by the calibration curve.

According to some examples of the second embodiment of the disclosure, the first detection method is based on a colorimetric method and the second detection method is based on a turbidimetric method. Accordingly, the controller 130 may be configured to:
control the sample preparation apparatus 110 to prepare a first test solution by using a sample to be tested and a colorimetric reagent, where the colorimetric reagent includes at least a coloring agent, and control the detection device 120 to detect the first test solution by using a colorimetric method, so as to acquire a colorimetric detection result for protein in the sample to be tested; and
control the sample preparation apparatus 110 to prepare a second test solution by using the sample to be tested and a turbidimetric reagent, and control the detection device 110 to detect the second test solution by using a turbidimetric method, so as to acquire a turbidimetric detection result for the protein in the sample to be tested, and select, on the basis of the colorimetric detection result, at least one of the colorimetric detection result and the turbidimetric detection result to determine a concentration of the protein in the sample to be tested.

Further, the controller 130 configured to select, on the basis of the colorimetric detection result, at least one of the colorimetric detection result and the turbidimetric detection result to determine a concentration of the protein in the sample to be tested, may include: the controller 130 configured to:
calculate the concentration of the protein in the sample to be tested only on the basis of the colorimetric detection result, if the colorimetric detection result is in a first predetermined range;
calculate the concentration of the protein in the sample to be tested only on the basis of the turbidimetric detection result, if the colorimetric detection result is in a second predetermined range.

Optionally, the controller 130 configured to select, on the basis of the colorimetric detection result, at least one of the colorimetric detection result and the turbidimetric detection result to determine a concentration of the protein in the sample to be tested, may further include: the controller 130 configured to:
calculate the concentration of the protein in the sample to be tested on the basis of the turbidimetric detection result and the colorimetric detection result, if the colorimetric detection result is in a third predetermined range.

For example, the second detection method is based on an immunoturbidimetric method, i.e., the turbidimetric reagent includes at least an antibody reagent. Accordingly, in some embodiments, the controller 130 configured to select, on the basis of the colorimetric detection result, at least one of the colorimetric detection result and the turbidimetric detection result to determine a concentration of the protein in the sample to be tested, may include: the controller 130 configured to: calculate the concentration of the protein in the sample to be tested only on the basis of the colorimetric detection result, if the colorimetric detection result is greater than a predetermined threshold value; and calculate the concentration of the protein in the sample to be tested only on the basis of the turbidimetric detection result, if the colorimetric detection result is not greater than the predetermined threshold value. In some other embodiments, the controller 130 configured to select, on the basis of the colorimetric detection result, at least one of the colorimetric detection result and the turbidimetric detection result to determine a concentration of the protein in the sample to be tested, may include: the controller 130 configured to: calculate the concentration of the protein in the sample to be tested only on the basis of the colorimetric detection result, if the colorimetric detection result is greater than a predetermined threshold value by a first difference value; calculate the concentration of the protein in the sample to be tested only on the basis of the turbidimetric detection result, if the colorimetric detection result is less than the predetermined threshold value by a second difference value; and calculate the concentration of the protein in the sample to be tested on the basis of the turbidimetric detection result and the colorimetric detection result, if an absolute difference between the colorimetric detection result and the predetermined threshold value is not greater than the first difference and the second difference.

As another example, the second detection method is based on a turbidimetric method on the basis of a protein precipitation reagent, i.e., the turbidimetric reagent includes at least a protein precipitation reagent. Accordingly, in some embodiments, the controller 130 configured to select, on the basis of the colorimetric detection result, at least one of the colorimetric detection result and the turbidimetric detection result to determine a concentration of the protein in the sample to be tested, may include: the controller 130 configured to: calculate the concentration of the protein in the sample to be tested only on the basis of the colorimetric detection result, if the colorimetric detection result is less than a predetermined threshold value; and calculate the concentration of the protein in the sample to be tested only on the basis of the turbidimetric detection result, if the colorimetric detection result is not less than the predetermined threshold value. In other embodiments, the controller 130 configured to select, on the basis of the colorimetric detection result, at least one of the colorimetric detection result and the turbidimetric detection result to determine a concentration of the protein in the sample to be tested, may include: the controller 130 configured to: calculate the concentration of the protein in the sample to be tested only on the basis of the colorimetric detection result, if the colorimetric detection result is less than a predetermined threshold value by a first difference value; calculate the concentration of the protein in the sample to be tested only on the basis of the turbidimetric detection result, if the colorimetric detection result is greater than the predetermined threshold value by a second difference value; and calculate the concentration of the protein in the sample to be tested on the basis of the turbidimetric detection result and the colorimetric detection result, if an absolute difference between the colorimetric detection result and the predetermined threshold value is not greater than the first difference and the second difference.

In embodiments of the disclosure, the first test solution and the second test solution may be prepared in the following several ways.

In a first manner of preparing a test solution, the controller 130 is configured to: control the sample preparation apparatus to prepare a first test solution by using a sample to be tested and a first detection reagent and control the detection device to detect the first test solution, and acquire a first detection result for protein in the sample to be tested; control the sample preparation apparatus to prepare a second test solution by using the first test solution and a second detection reagent, and control the detection device to detect the second test solution, so as to acquire a second detection result for the protein in the sample to be tested. Alternatively, the controller 130 is configured to: control the sample preparation apparatus to prepare a first test solution by using a sample to be tested and a first detection reagent and control the detection device to detect the first test solution, and acquire a first detection result for protein in the sample to be tested; if determine, on the basis of the first detection result, a protein detection on the basis of the second detection method is required to be performed for the sample to be tested, then control the sample preparation apparatus to prepare a second test solution by using the first test solution and a second detection reagent, and control the detection device to detect the second test solution, and acquire a second detection result for the protein in the sample to be tested. Thus, the protein concentration in the sample to be tested can be determined on the basis of the first detection result and the second detection result.

For example, the controller 130 configured to control the sample preparation apparatus 110 to prepare a first test solution by using a sample to be tested and a colorimetric reagent, includes: the controller 130 configured to control the sample preparation apparatus 110 to mix at least a portion of the sample to be tested with the colorimetric reagent to prepare the first test solution; and the controller 130 configured to control the sample preparation apparatus 110 to prepare a second test solution by using the sample to be tested and a turbidimetric reagent, includes: the controller 130 configured to control the sample preparation apparatus 110 to mix at least a portion of the first test solution with the turbidimetric reagent to prepare the second test solution. Here, the sample analyzer shown in FIG. 2 is used as an example to describe the first manner of preparing a test solution. The controller 130 configured to control the sample preparation apparatus 110 to prepare a first test solution by using a sample to be tested and a colorimetric reagent, includes: the controller 130 configured to: control the reagent dispensing mechanism 114 to add the colorimetric reagent in the reagent holding component 113 to a first reaction cup located in the reaction component 115, then control the sample dispensing mechanism 112 to aspirate a portion of a sample to be tested, particularly, an urine sample, from the container 10, and to add the portion of the sample to be tested to the first reaction cup to which the colorimetric reagent has been added, so as to obtain the first test solution. The controller 130 configured to control the sample preparation apparatus 110 to prepare a second test solution by using the sample to be tested and a turbidimetric reagent, includes: the controller 130 configured to: control the reagent dispensing mechanism 114 to add a turbidimetric reagent in the reagent holding component 113 to a second reaction cup located in the reaction component 115, and control a test solution transferring component to transfer a portion of the first test solution in the first reaction cup to the second reaction cup to which the turbidimetric reagent has been added, so as to prepare the second test solution.

In a second manner of preparing a test solution, the controller 130 is configured to: control the sample preparation apparatus to prepare a first test solution by using a first portion of a sample to be tested and a first detection reagent and control the detection device to detect the first test solution, and acquire a first detection result for protein in the sample to be tested; control the sample preparation apparatus to prepare a second test solution by using a second portion of the sample to be tested and a second detection reagent, and control the detection device to detect the second test solution, and acquire a second detection result for the protein in the sample to be tested. Alternatively, the controller 130 is configured to: control the sample preparation apparatus to prepare a first test solution by using a first portion of a sample to be tested and a first detection reagent and control the detection device to detect the first test solution, and acquire a first detection result for protein in the sample to be tested; if determine, on the basis of the first detection result, a protein detection on the basis of a second detection method is required to be performed for the sample to be tested, then control the sample preparation apparatus to prepare a second test solution by using a second portion of the sample to be tested and a second detection reagent, and control the detection device to detect the second test solution, and acquire a second detection result for the protein in the sample to be tested. Thus, the protein concentration in the sample to be tested can be determined on the basis of the first detection result and the second detection result.

It is understood here that the first portion of the sample to be tested is a completely different portion from the second portion of the sample to be tested. For example, the first portion of the sample to be tested and the second portion of the sample to be tested are from different portions of the sample to be tested in a same sample container. As another example, the first portion of the sample to be tested and the second portion of the sample to be tested are from samples to be tested in different sample containers.

For example, the controller 130 configured to control the sample preparation apparatus 110 to prepare a first test solution by using a sample to be tested and a colorimetric reagent, includes: the controller 130 configured to control the sample preparation apparatus 110 to mix at least a portion of the sample to be tested with the colorimetric reagent to prepare the first test solution; and the controller 130 configured to control the sample preparation apparatus 110 to prepare a second test solution by using the sample to be tested and a turbidimetric reagent, includes: the controller 130 configured to control the sample preparation apparatus 110 to mix at least another portion of the sample to be tested with the turbidimetric reagent to prepare the second test solution.

Also, the sample analyzer shown in FIG. 2 is used as an example to describe the second manner of preparing a test solution. The controller 130 configured to control the sample preparation apparatus 110 to prepare a first test solution by using a sample to be tested and a colorimetric reagent, includes: the controller 130 configured to: control the reagent dispensing mechanism 114 to add the colorimetric reagent in the reagent holding component 113 to a first reaction cup located in the reaction component 115, then control the sample dispensing mechanism 112 to aspirate a portion of the sample to be tested, particularly, urine sample, from the container 10, and to add the portion of the sample to be tested to the first reaction cup to which the colorimetric reagent has been added, so as to obtain the first test solution. The controller 130 configured to control the sample preparation apparatus 110 to prepare a second test solution by using the sample to be tested and a turbidimetric reagent, includes: the controller 130 configured to: control the reagent dispensing mechanism 114 to add the turbidimetric reagent in the reagent holding component 113 to a second reaction cup located in the reaction component 115, then control the sample dispensing mechanism 112 to aspirate another portion of the sample to be tested from the container 10 again or aspirate a portion of the sample to be tested from another container containing the sample to be tested of the same subject, and to add the portion of the sample to be tested to the second reaction cup to which the turbidimetric reagent has been added, so as to obtain the second test solution.

According to some other examples of the first embodiment of the disclosure, the first detection method is based on a turbidimetric method and the second detection method is based on a colorimetric method. Accordingly, the controller 130 may be configured to:
control the sample preparation apparatus 110 to prepare a first test solution by using a sample to be tested and a turbidimetric reagent, and control the detection device 120 to detect the first test solution by using a turbidimetric method, so as to acquire a turbidimetric detection result for protein in the sample to be tested;
determine, on the basis of the turbidimetric detection result, whether to perform a colorimetric test;
if no, then determine a concentration of the protein in the sample to be tested on the basis of the colorimetric detection result (e.g., determine the concentration of the protein in the sample to be tested only on the basis of the colorimetric detection result); and
if yes, then control the sample preparation apparatus 110 to prepare a second test solution by using the sample to be tested and a colorimetric reagent, and control the detection device 120 to detect the second test solution by using a colorimetric method, so as to acquire a colorimetric detection result for the protein in the sample to be tested, and determine a concentration of the protein in the sample to be tested on the basis of the colorimetric detection result and optionally the turbidimetric detection result, (e.g., determine the concentration of the protein in the sample to be tested only on the basis of the colorimetric detection result, or determine the concentration of the protein in the sample to be tested on the basis of both the turbidimetric detection result and the colorimetric detection result), where the colorimetric reagent includes at least a coloring agent.

Further, the controller 130 configured to determine, on the basis of the turbidimetric detection result, whether to perform a colorimetric test, may include: the controller 130 configured to:
determine that the colorimetric test is not required to be performed, if the turbidimetric detection result is in a first predetermined range; and
determine that the colorimetric test is required to be performed, if the turbidimetric detection result is in a second predetermined range that is different from the first predetermined range.

In some embodiments, the first detection method is based on an immunoturbidimetric method, i.e., the turbidimetric reagent includes at least an antibody reagent. In this case, the sample analyzer is especially suitable for detecting albumin in a biological sample, preferably in urine. Accordingly, the controller 130 configured to determine, on the basis of the turbidimetric detection result, whether to perform a colorimetric test, may include: the controller 130 configured to:
determine that the colorimetric test is not required to be performed, if the turbidimetric detection result is less than a predetermined threshold value; and
determine that the colorimetric test is required to be performed, if the turbidimetric detection result is not less than the predetermined threshold value.

Here, the sample analyzer is especially suitable for detecting albumin or microalbumin in urine. In this case, the albumin concentration detection upper limit of the colorimetric method is greater than the albumin concentration detection upper limit of the immunoturbidimetric method, and the albumin concentration detection lower limit of the colorimetric method is greater than the albumin concentration detection lower limit of the immunoturbidimetric method.

The predetermined threshold value here may be determined empirically or may be determined according to the albumin concentration detection upper limit of the immunoturbidimetric method and the albumin concentration detection lower limit of the colorimetric method, for example, may be selected to be substantially greater than the albumin concentration detection lower limit of the colorimetric method and substantially less than the albumin concentration detection upper limit of the turbidimetric method.

Alternatively, in some other embodiments, the first detection method is based on a turbidimetric method on the basis of a protein precipitation reagent, i.e., the turbidimetric reagent includes at least a protein precipitation reagent. In this case, the sample analyzer is especially suitable for detecting total protein in a biological sample, preferably in urine. Accordingly, the controller 130 configured to determine, on the basis of the turbidimetric detection result, whether to perform a colorimetric test, may include: the controller 130 configured to:
determine that the colorimetric test is not required to be performed, if the turbidimetric detection result is greater than a predetermined threshold value; and
determine that the colorimetric test is required to be performed, if the turbidimetric detection result is not greater than the predetermined threshold value.

Here, the sample analyzer is especially suitable for detecting total protein in urine. In this case, the total protein concentration detection upper limit of the colorimetric method is less than the total protein concentration detection upper limit of the turbidimetric method on the basis of the protein precipitation reagent, and the total protein concentration detection lower limit of the colorimetric method is less than the total protein concentration detection lower limit of the turbidimetric method on the basis of the protein precipitation reagent.

The predetermined threshold value here may be determined empirically or may be determined according to the total protein concentration detection upper limit of the colorimetric method and the total protein concentration detection lower limit of the turbidimetric method on the basis of the protein precipitation reagent, for example, may be selected to be substantially greater than the total protein concentration detection lower limit of the turbidimetric method on the basis of the protein precipitation reagent and substantially less than the total protein concentration detection upper limit of the colorimetric method.

In some embodiment, the predetermined threshold value may be determined based on light intensity signals or absorbance signals during reaction of the sample to be tested with the turbidimetric reagent or based on a reaction curve of the sample to be tested with the turbidimetric reagent. For example, the predetermined threshold value may be determined based on a protein concentration converted from the absorbance signals through a calibration curve, or may be determined based on a slope of a certain curve segment of the reaction curve.

In other embodiments, the predetermined threshold value may also be determined by the calibration curve.

According to some other examples of the second embodiment of the disclosure, the first detection method is based on a turbidimetric method and the second detection method is based on a colorimetric method. Accordingly, the controller 130 may be configured to:
control the sample preparation apparatus 110 to prepare a first test solution by using a sample to be tested and a turbidimetric reagent, and control the detection device 120 to detect the first test solution by using a colorimetric method, so as to acquire a colorimetric detection result for protein in the sample to be tested; and
control the sample preparation apparatus 110 to prepare a second test solution by using the sample to be tested and a colorimetric reagent, control the detection device 120 to detect the second test solution by using a colorimetric method, so as to acquire a colorimetric detection result for the protein in the sample to be tested, where the colorimetric reagent includes at least a coloring agent, and select, on the basis of the turbidimetric detection result, at least one of the colorimetric detection result and the turbidimetric detection result to determine a concentration of the protein in the sample to be tested.

Further, the controller 130 configured to select, on the basis of the turbidimetric detection result, at least one of the colorimetric detection result and the turbidimetric detection result to determine a concentration of the protein in the sample to be tested, may include: the controller 130 configured to:
calculate the concentration of the protein in the sample to be tested only on the basis of the turbidimetric detection result, if the turbidimetric detection result is in a first predetermined range;
calculate the concentration of the protein in the sample to be tested only on the basis of the colorimetric detection result, if the turbidimetric detection result is in a second predetermined range.

Optionally, the controller 130 configured to select, on the basis of the turbidimetric detection result, at least one of the colorimetric detection result and the turbidimetric detection result to determine a concentration of the protein in the sample to be tested, may further include: the controller 130 configured to:
calculate the concentration of the protein in the sample to be tested on the basis of the turbidimetric detection result and the colorimetric detection result, if the turbidimetric detection result is in a third predetermined range.

For example, the first detection method is based on an immunoturbidimetric method, i.e., the turbidimetric reagent includes at least an antibody reagent. Accordingly, in some embodiments, the controller 130 configured to select, on the basis of the turbidimetric detection result, at least one of the colorimetric detection result and the turbidimetric detection result to determine a concentration of the protein in the sample to be tested, includes: the controller 130 configured to: calculate the concentration of the protein in the sample to be tested only on the basis of the turbidimetric detection result, if the turbidimetric detection result is less than a predetermined threshold value; and calculate the concentration of the protein in the sample to be tested only on the basis of the colorimetric detection result, if the turbidimetric detection result is not less than the predetermined threshold value. In other embodiments, the controller 130 configured to select, on the basis of the turbidimetric detection result, at least one of the colorimetric detection result and the turbidimetric detection result to determine a concentration of the protein in the sample to be tested, includes: the controller 130 configured to: calculate the concentration of the protein in the sample to be tested only on the basis of the turbidimetric detection result, if the turbidimetric detection result is less than a predetermined threshold value by a first difference value; calculate the concentration of the protein in the sample to be tested only on the basis of the colorimetric detection result, if the turbidimetric detection result is greater than the predetermined threshold value by a second difference value; and calculate the concentration of the protein in the sample to be tested on the basis of the turbidimetric detection result and the colorimetric detection result, if an absolute difference between the turbidimetric detection result and the predetermined threshold value is not greater than the first difference and the second difference.

As another example, the first detection method is based on a turbidimetric method on the basis of a protein precipitation reagent, i.e., the turbidimetric reagent includes at least a protein precipitation reagent. Accordingly, in some embodiments, the controller 130 configured to select, on the basis of the turbidimetric detection result, at least one of the colorimetric detection result and the turbidimetric detection result to determine a concentration of the protein in the sample to be tested, includes: the controller 130 configured to: calculate the concentration of the protein in the sample to be tested only on the basis of the turbidimetric detection result, if the turbidimetric detection result is greater than a predetermined threshold value; and calculate the concentration of the protein in the sample to be tested only on the basis of the colorimetric detection result, if the turbidimetric detection result is not greater than the predetermined threshold value. In other embodiments, the controller 130 configured to select, on the basis of the turbidimetric detection result, at least one of the colorimetric detection result and the turbidimetric detection result to determine a concentration of the protein in the sample to be tested, includes: the controller 130 configured to: calculate the concentration of the protein in the sample to be tested only on the basis of the turbidimetric detection result, if the turbidimetric detection result is greater than a predetermined threshold value by a first difference value; calculate the concentration of the protein in the sample to be tested only on the basis of the colorimetric detection result, if the turbidimetric detection result is less than the predetermined threshold value by a second difference value; and calculate the concentration of the protein in the sample to be tested on the basis of the turbidimetric detection result and the colorimetric detection result, if an absolute difference between the turbidimetric detection result and the predetermined threshold value is not greater than the first difference and the second difference.

In some embodiments, the controller 130 configured to control the sample preparation apparatus 110 to prepare a first test solution by using a sample to be tested and a turbidimetric reagent, may include: the controller 130 configured to control the sample preparation apparatus 110 to mix at least a portion of the sample to be tested with the turbidimetric reagent to prepare the first test solution; and the controller 130 configured to control the sample preparation apparatus 110 to prepare a second test solution by using the sample to be tested and a colorimetric reagent, includes: the controller 130 configured to control the sample preparation apparatus 110 to mix at least a portion of the first test solution with the colorimetric reagent to prepare the second test solution.

In some other embodiments, the controller 130 configured to control the sample preparation apparatus 110 to prepare a first test solution by using a sample to be tested and a turbidimetric reagent, may include: the controller 130 configured to control the sample preparation apparatus 110 to mix at least a portion of the sample to be tested with the turbidimetric reagent to prepare the first test solution; and the controller 130 configured to control the sample preparation apparatus 110 to prepare a second test solution by using the sample to be tested and a colorimetric reagent, includes: the controller 130 configured to control the sample preparation apparatus 110 to mix at least another portion of the sample to be tested with the colorimetric reagent to prepare the second test solution.

In some embodiments, the controller 130 configured to determine or calculate the concentration of the protein in the sample to be tested on the basis of the turbidimetric detection result and the colorimetric detection result, may include: the controller 130 configured to calculate a weighted sum or weighted average of the turbidimetric detection result and the colorimetric detection result.

In embodiments of the disclosure, the colorimetric detection result may be a protein concentration, or may be an intermediate value, such as an absorbance, related to the protein concentration obtained during the colorimetric detection. Similarly, the turbidimetric detection result may be a protein concentration, or may be an intermediate value, such as an absorbance, related to the protein concentration obtained during the turbidimetric detection.

In some embodiments, the detection device 120 is configured as a light-measuring device for measuring absorbance.

As some embodiments, the light-measuring device is configured to measure absorbance of the first test solution or second test solution at least at a primary wavelength, where the primary wavelength is the wavelength at or near the peak of the target absorption curve corresponding to the reaction of protein with the detection reagent.

Further, the light-measuring device is configured to measure absorbance of the first test solution or second test solution at a primary wavelength and a secondary wavelength, where the primary wavelength is the wavelength at or near the peak of the target absorption curve corresponding to the reaction of protein with the detection reagent, and the secondary wavelength is the wavelength near the peak of the interference absorption curve of interfering substances (e.g., alkaline substances in the case of the colorimetric method) during the reaction of protein with the detection reagent. Thus, a more accurate protein detection result can be obtained by subtracting the absorbance of the interfering substances from the absorbance detected at the primary wavelength.

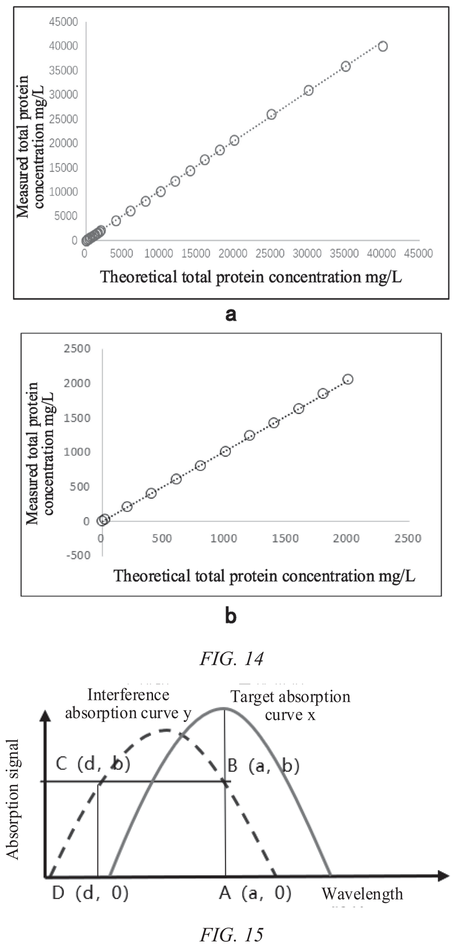

For example, FIG. 15 shows a target absorption curve x and an interference absorption curve y. Taking the wavelength a at the peak of the target absorption curve or the wavelength near the peak of the target absorption curve as the primary wavelength; The absorption signal on the interference absorption curve corresponding to A is b (this point is point B) and the point on the interference absorption curve symmetrical to point B with respect to the peak is point C, i.e. the other point on the interference absorption curve where the absorption signal is b, the wavelength corresponding to the point C is d, then d or the wavelength near d is taken as the secondary wavelength. This can ensure the signal intensity detected at the primary wavelength as much as possible, and can also remove superfluous signals caused by interference.

This embodiment is especially suitable for protein detection, especially albumin detection, based on the colorimetric method. For example, since commonly used coloring agents such as bromocresol green, bromophenol purple itself are also acid-base indicators, when urine is slightly alkaline, color changes can occur, interfering with albumin detection.

An embodiment of the disclosure also provides a protein analysis method for a sample analyzer including a sample preparation apparatus, a detection device, and a controller. In particular, the sample analyzer is constructed as the sample analyzer 100 described above.

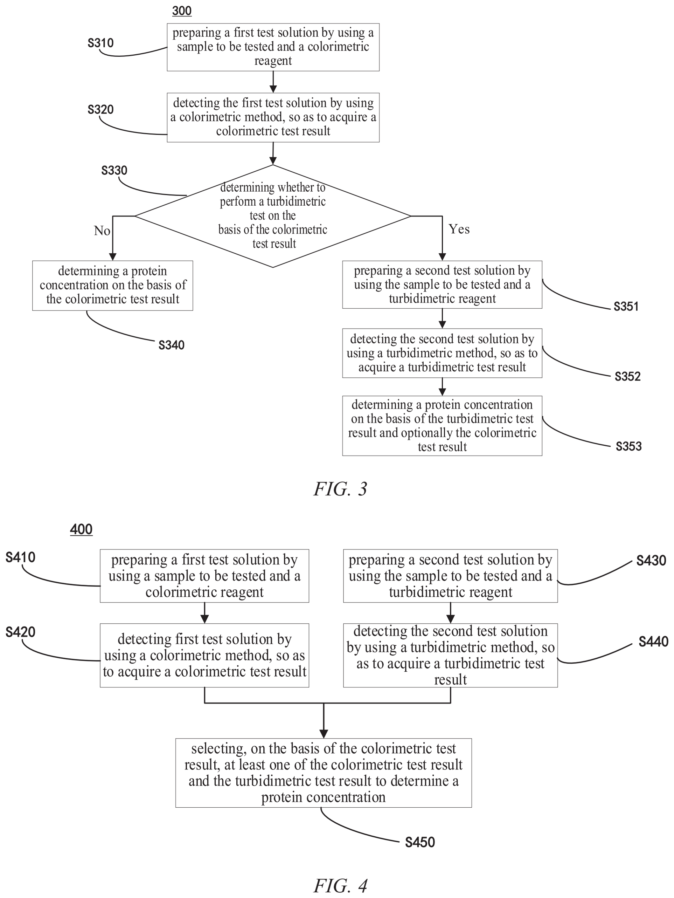

FIG. 3 is a schematic flowchart of a protein analysis method 300 provided according to a first example of the disclosure. The protein analysis method 300 includes the following steps:
S310, controlling, by the controller, the sample preparation apparatus to prepare a first test solution by using a sample to be tested and a colorimetric reagent, where the colorimetric reagent includes at least a coloring agent;
S320, controlling, by the controller, the detection device to detect the first test solution by using a colorimetric method, so as to acquire a colorimetric detection result for protein in the sample to be tested;
S330, determining, by the controller, on the basis of the colorimetric detection result, whether to perform a turbidimetric test;
S340, if no, then determining, by the controller, a concentration of the protein in the sample to be tested on the basis of the colorimetric detection result;
if yes, then performing the following sub-steps by the controller:
   S351, controlling the sample preparation apparatus to prepare a second test solution by using the sample to be tested and a turbidimetric reagent,
   S352, controlling the detection device to detect the second test solution by using a turbidimetric method, so as to acquire a turbidimetric detection result for the protein in the sample to be tested, and
   S353, determining a concentration of the protein in the sample to be tested on the basis of the turbidimetric detection result and optionally the colorimetric detection result.

FIG. 4 is a schematic flowchart of a protein analysis method 400 provided according to a second example of the disclosure. The protein analysis method 400 includes the following steps:
S410, controlling, by the controller, the sample preparation apparatus to prepare a first test solution by using a sample to be tested and a colorimetric reagent, where the colorimetric reagent includes at least a coloring agent;
S420, controlling by the controller, the detection device to detect the first test solution by using a colorimetric method, so as to acquire a colorimetric detection result for protein in the sample to be tested;
S430, controlling, by the controller, the sample preparation apparatus to prepare a second test solution by using the sample to be tested and a turbidimetric reagent;
S440, controlling, by the controller, the detection device to detect the second test solution by using a turbidimetric method, so as to acquire a turbidimetric detection result for the protein in the sample to be tested; and
S450, selecting, by the controller, on the basis of the colorimetric detection result, at least one of the colorimetric detection result and the turbidimetric detection result to determine a concentration of the protein in the sample to be tested.

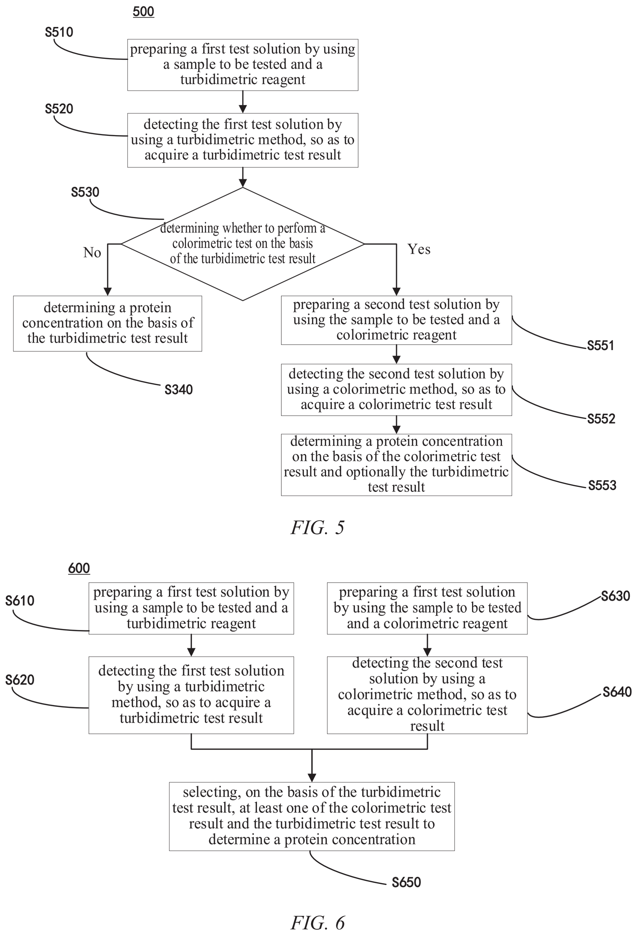

FIG. 5 is a schematic flowchart of a protein analysis method 500 provided according to a third example of the disclosure. The protein analysis method 500 includes the following steps:
S510, controlling, by the controller, the sample preparation apparatus to prepare a first test solution by using the sample to be tested and a turbidimetric reagent;
S520, controlling, by the controller, the detection device to detect the first test solution by using a turbidimetric method, so as to acquire a turbidimetric detection result for protein in the sample to be tested;
S530, determining, by the controller, on the basis of the turbidimetric detection result, whether to perform a colorimetric test;
S540, if no, then determining, by the controller, a concentration of the protein in the sample to be tested on the basis of the colorimetric detection result;
if yes, then performing the following sub-steps by the controller:
   S551, controlling the sample preparation apparatus to prepare a second test solution by using the sample to be tested and a colorimetric reagent, where the colorimetric reagent includes at least a coloring agent;
   S552, controlling the detection device to detect the second test solution by using a colorimetric method, so as to acquire a colorimetric detection result for the protein in the sample to be tested;
   S553, determining a concentration of the protein in the sample to be tested on the basis of the colorimetric detection result and optionally the turbidimetric detection result.

FIG. 6 is a schematic flowchart of a protein analysis method 600 provided according to a fourth example of the disclosure. The protein analysis method 600 includes the following steps:
S610, controlling, by the controller, the sample preparation apparatus to prepare a first test solution by using a sample to be tested and a turbidimetric reagent;
S620, controlling, by the controller, the detection device to detect the first test solution by using a turbidimetric method, so as to acquire a turbidimetric detection result for protein in the sample to be tested;
S630, controlling, by the controller, the sample preparation apparatus to prepare a second test solution by using the sample to be tested and a colorimetric reagent, where the colorimetric reagent includes at least a coloring agent;
S640, controlling, by the controller, the detection device to detect the second test solution by using a colorimetric method, so as to acquire a colorimetric detection result for the protein in the sample to be tested; and
S650, selecting, by the controller, on the basis of the turbidimetric detection result, at least one of the colorimetric detection result and the turbidimetric detection result to determine a concentration of the protein in the sample to be tested.

It will be appreciated that various embodiments of the sample analyzer 100 described above and the advantages thereof can be correspondingly adapted to the protein analysis methods provided in the embodiments of the disclosure.

An embodiment of the disclosure also relates to a protein analysis reagent. This protein analysis reagent includes a colorimetric reagent and a turbidimetric reagent, where the colorimetric reagent includes a coloring agent and the turbidimetric reagent includes an antibody reagent or a protein precipitation reagent. The protein analysis reagent provided in the embodiment of the disclosure is especially applicable to the sample analyzer and the protein analysis methods provided in the embodiments of the disclosure, and can effectively extend the detection range of a single protein assay, it can obtain protein concentrations within an extremely wide range through one single assay.

In the embodiments of the disclosure, the coloring agent refers to a substance that is capable of reacting with protein, such as albumin, to cause a spectral change. Here, the reaction of the coloring agent with protein, such as albumin, can produce a color change visible to the naked eye, or can produce a change that is not visible to the naked eye but cause a change in the absorption spectrum, for example, a change in the ultraviolet-visible absorption spectrum.

In some embodiments, the coloring agent may be an acid-base indicator. For example, the coloring agent may be selected from one of pentamethoxy red, methylquinoline red, bromocresol green, and bromocresol purple. For another example, the coloring agent may be selected from the group consisting of azo compounds (compounds having a nitrogen-nitrogen double bond, such as methyl orange), sulfonephthalein compounds (compounds having a phenolic sulfo group and a phenolic hydroxyl group, e.g. bromocresol green), phenolphthalein compounds (phenolphthalein and derivatives thereof, e.g. phenolphthalein, thymol blue, α-naphtholphthalein), anthraquinone compounds (anthracene compounds having carbonyl groups at the C9 and C10 positions, such as alizarin), nitrophenol compounds (compounds having a phenolic nitro group and a phenolic hydroxyl group, such as p-nitrophenol) and any combination thereof (e.g. Evans blue, i.e. azo + sulfonephthalein).

In some other embodiments, the coloring agent may be a substance that reacts with the peptide bond of protein. For example, copper ions react with the peptide bond of protein under alkaline conditions to form a purple-red complex.

In some yet other embodiments, the coloring agent may be a substance that reacts with the tyrosine residues and tryptophan residues of protein, such as a phosphomolybdic acid-phosphotungstic acid reagent.

In some still other embodiments, the coloring agent may be an anionic dye that binds to the amino groups of protein for example under acidic conditions, e.g., amino black, ponceau S, Coomassie brilliant blue, pyrogallol red molybdenum, etc.

In the embodiments of the disclosure, the antibody reagent refers to an antibody that is capable of specifically binding to protein, such as albumin, to produce turbidity. For example, the antibody reagent includes a solid-phase carrier with an antibody attached to a surface thereof, where the solid-phase carrier preferably is latex microspheres. The antibody also includes a recombinantly expressed artificial antibody, a nucleic acid fragment that functions as an antibody (an aptamer), and an antibody fragment that retains an antibody binding capability, such as a Fab fragment.

In the embodiments of the disclosure, the protein precipitation reagent refers to a substance that is capable of precipitating protein, especially total protein, to produce turbidity. For example, the protein precipitation reagent is sulfosalicylic acid, trichloroacetic acid, or the like.

In some embodiments, the colorimetric reagent further includes a turbidity-enhancing reagent including a hydrophilic polymer, especially polyethylene glycol 6000; the turbidimetric reagent includes an antibody reagent.

In some embodiments, the coloring agent and the turbidity-enhancing reagent are mixed and packaged as a first reagent R1, while the turbidimetric reagent including the antibody reagent is packaged as a second reagent R2. For example, the protein analysis reagent of this embodiment is used to detect protein in a sample to be tested in the following ways: preparing a first test solution by using the sample to be tested and the first reagent R1; detecting the first test solution by using a colorimetric method, so as to acquire a colorimetric detection result for the protein in the sample to be tested; calculating a concentration of the protein on the basis of the colorimetric detection result, if the colorimetric detection result is in a first predetermined range; preparing a second test solution by using the first test solution and the second reagent R2, and detecting the second test solution by using a turbidimetric method, if the colorimetric detection result is in a second predetermined range, for example not in the first predetermined range, so as to acquire a turbidimetric detection result for the protein in the sample to be tested and calculate a concentration of the protein on the basis of the turbidimetric detection result.

In this case, both the first reagent R1 and the second reagent R2 may react with analyte, i.e. the protein, especially albumin, in the sample. For the analyte, the first reagent R1 and the second reagent R2 have different detection ranges, in particular the first reagent R1 has a relatively high concentration detection upper limit, and the second reagent R2 has a relatively concentration detection low lower limit.

For example, during detection, the first reagent R1 may first react with the analyte in the sample to obtain a reaction solution including an analyte-coloring agent complex. When the concentration of the analyte is relatively high, for example, when the concentration of the analyte is within the detection range of the first reagent R1, the first reagent R1 is capable of providing a sufficient reaction signal for calculating the concentration of the analyte (e.g., after mixing the first reagent R1 with the sample, the absorbance change signal S1 of the reaction solution before and after the mixing is read, and the concentration of the analyte is calculated based on the absorbance change signal S1) signal, and when the concentration of the analyte in the sample is relatively low, for example, when the concentration of the analyte is not within the detection range of the first reagent R1, R1 is unable to provide a sufficient reaction signal, then the second reagent R2 is added to the reaction solution formed by mixing the first reagent R1 with the sample, and the antibody reagent contained in the second reagent R2 binds to the analyte-coloring agent complex in the reaction solution, such that a reaction signal is obtained through the second reagent R2 (or through the action of the first reagent R1 in combination with the second reagent R2), thereby obtaining a concentration of the analyte (e.g. after mixing the second reagent R2 with the reaction solution containing the analyte-coloring agent complex, the absorbance change signal S2 of the reaction solution before and after the mixing is read, and the concentration of the analyte is calculated based on the absorbance change signal S2).

Here, a specific exemplary process for using the protein analysis reagent of this embodiment for detecting albumin in a sample to be tested according to the disclosure is as follows: the first reagent R1 containing the coloring agent and the turbidity-enhancing reagent, the sample, the second reagent R2 containing the antibody reagent, are added sequentially to a reaction cup and the absorbance change of the reaction solution is measured. When the albumin concentration in the sample is not abnormally high, the coloring agent in the first reagent R1 is not sufficient to produce a sufficient color change, the albumin in the sample binds to the antibody reagent in the second reagent R2 to produce turbidity, a concentration detection signal is obtained, and then the concentration detection signal is compared to a corresponding standard curve to obtain an albumin concentration result; when the albumin concentration in the sample is sufficiently high, the color change produced by the coloring agent in the first reagent R1 and the albumin in the sample is sufficient to be discerned by the sample analyzer, which reads absorbance information at a specific wavelength, then compares the absorbance information with a corresponding standard curve to obtain albumin concentration information. Therefore, the low detection lower limit of the turbidimetric method and the high detection upper limit of the colorimetric method are combined to broaden the detection range and solve the inadequacy of existing detection means.

In some other embodiments, the coloring agent, the turbidity-enhancing reagent, and the turbidimetric reagent including the antibody reagent are separately and independently packaged, i.e., the coloring agent is packaged as a first reagent R1, the turbidity-enhancing reagent is packaged as a second reagent R2, and the antibody reagent is packaged as a third reagent R3. For example, the protein analysis reagent of this embodiment is used to detect protein in a sample to be tested in the following ways: preparing a first test solution by using the sample to be tested and the first reagent R1; detecting the first test solution by using a colorimetric method, so as to acquire a colorimetric detection result for the protein in the sample to be tested; calculating a concentration of the protein on the basis of the colorimetric detection result, if the colorimetric detection result is in a first predetermined range; preparing a second test solution by using the first test solution and the second reagent R2 and the third reagent R3, and detecting the second test solution by using a turbidimetric method, if the colorimetric detection result is in a second predetermined range, for example not in the first predetermined range, so as to acquire a turbidimetric detection result for the protein in the sample to be tested and calculate a concentration of the protein on the basis of the turbidimetric detection result.

The technical solution of the prior art and the technical solution of the disclosure are verified below with reference to some examples, each of which is implemented on a Mindray BS-2000 Biochemical Analyzer.

### Example 1 Detection of albumin in urine based on a colorimetric method according to the prior art

### 1.1 Colorimetric reagent

### Bromocresol green

### BRIJ35

1.2 Colorimetric method-based detection method: 200 uL of the colorimetric reagent was added to a reaction cup, the absorbance S0 was read at a primary wavelength of 605 nm and a secondary wavelength of 700 nm (where S0 = absorbance at 605 nm - absorbance at 700 nm), then 2 uL of a urine sample was added to the reaction cup and the absorbance S1 was read after about 3 minutes at the primary wavelength of 605 nm and the secondary wavelength of 700 nm (where S1 = absorbance at 605 nm - absorbance at 700 nm). The final obtained signal value is S = S1-SO*200/(200+2) and the albumin concentration value corresponding to the obtained signal value was determined by a calibration curve.

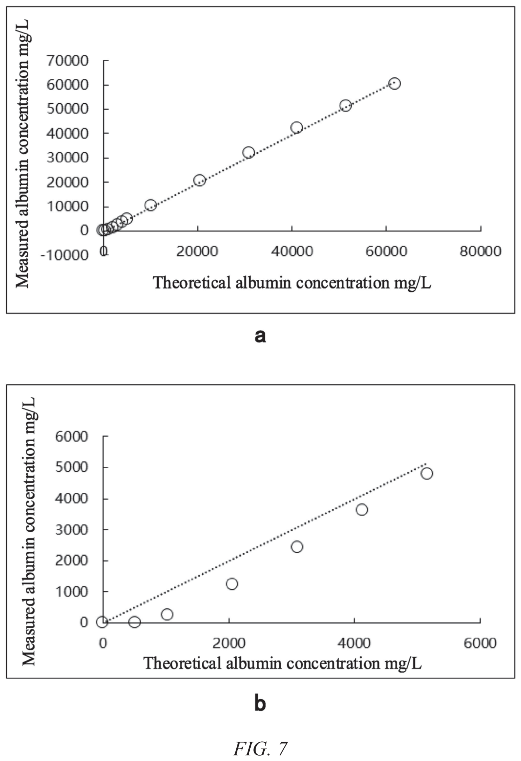

1.3 Multiple urine samples with known albumin concentrations were detected on the Mindray BS-2000 Biochemical Analyzer using the colorimetric reagent and colorimetric method-based detection method described above to obtain measured albumin concentrations based on the colorimetric method. FIG. 7 shows a linear relationship between the known albumin concentrations (theoretical albumin concentrations) and the measured albumin concentrations based on the colorimetric method of the multiple urine samples. FIG. 7a shows a linear relationship between the known albumin concentrations and the measured albumin concentrations based on the colorimetric method of all urine samples among these multiple urine samples, while FIG. 7b shows a linear relationship between the known albumin concentrations and the measured albumin concentrations based on the colorimetric method of the urine samples with relatively low albumin concentrations.

As can be seen in FIG. 7a, the colorimetric method for detecting albumin in urine has a sufficiently high upper limit of linear detection, which can reach 60000 mg/L. However, as shown in Fig. 7b, the measured albumin concentration deviates significantly from the theoretical albumin concentration when the albumin concentration is low, and accurate albumin concentration values cannot be obtained at albumin concentrations less than 250 mg/L (due to excessively low signal), and thus fails to meet the detection requirements for urine samples (for urine albumin detection, more than 60% of the samples have an albumin concentration less than 200 mg/L).

### Example 2 Detection of albumin in urine based on an immunoturbidimetric method according to the prior art

### 2.1 Turbidimetric reagent

### First reagent R1: containing PEG6000, NaCl and Tris buffer

### Second reagent R2: containing ALB (albumin) antiserum and Tris buffer.

2.2 Turbidimetric method-based detection method: 180 uL of the first reagent R1 and 6 uL of a urine sample were added sequentially to a reaction cup, the absorbance S0 was read at a wavelength of 340 nm after incubation for about 3 minutes, then 30 uL of the second reagent R2 was further added to the reaction cup, and the absorbance S1 was read at the wavelength of 340 nm after about 4.5 minutes. The final obtained signal value is S = S1-SO*(180+6)/(180+6+30) and the albumin concentration value corresponding to the obtained signal value was determined by a calibration curve.

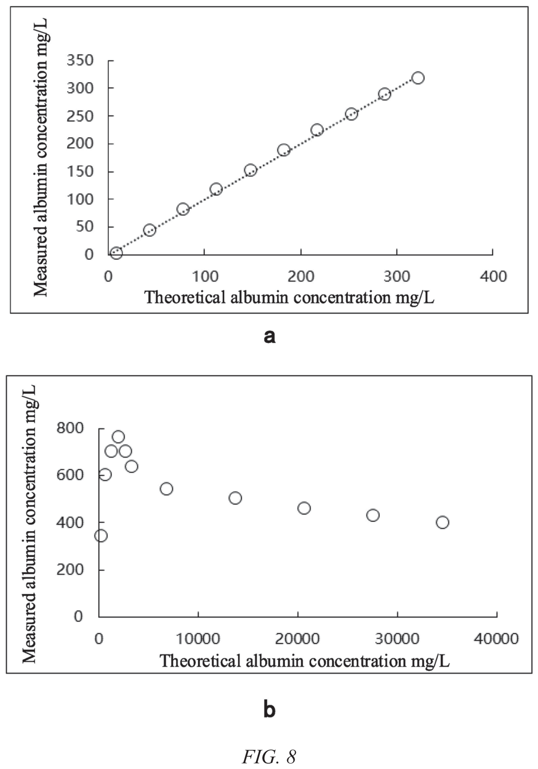

2.3 Multiple urine samples with known albumin concentrations were detected on the Mindray BS-2000 Biochemical Analyzer using the turbidimetric reagent and turbidimetric method-based detection method described above to obtain measured albumin concentrations based on the turbidimetric method. FIG. 8 shows a linear relationship between the known albumin concentrations (theoretical albumin concentrations) and the measured albumin concentration based on the turbidimetric method of the multiple urine samples. FIG. 8a shows a linear relationship between the known albumin concentrations and the measured albumin concentrations based on the turbidimetric method of the urine samples with relatively low albumin concentrations, while FIG. 8b shows a linear relationship between the known albumin concentrations and the measured albumin concentrations based on the turbidimetric method of all urine samples among these multiple urine samples.

As shown in FIG. 8a, the turbidimetric method has a better linearity in the low albumin concentration range (less than 300 mg/L). However, as shown in FIG. 8b, albumin concentrations cannot be accurately obtained at relatively high albumin concentrations, and even a hook effect occurs (> 2000 mg/L).

### Example 3 Detection of albumin in urine using a combination of a colorimetric method and an immunoturbidimetric method according to the disclosure

### 3.1 Protein analysis reagent according to the disclosure

### First reagent R1: containing PEG6000, bromocresol green and BRIJ35

### Second reagent R2: containing ALB (albumin) antiserum and Tris buffer

### 3.2 Calibration method

A series of calibrators with known albumin concentrations or assigned to determined albumin concentrations were used as samples, and the calibrators are classified into high-concentration calibrators (with albumin concentration above a predetermined threshold value) and low-concentration calibrators (with albumin concentration not above the predetermined threshold value) by the predetermined threshold value.

For each high concentration calibrator: 100 uL of the first reagent R1 was added to the reaction cup; the absorbance S0 was read at a wavelength of 605 nm; then 6 uL of this high concentration calibrator was further added to the reaction cup; the absorbance S1H was read at the wavelength of 605 nm; finally the signal value was calculated: SH = S1H-SO*100/(100+6).

A high-range calibration curve was fitted using the albumin concentration and signal value SH of each high-concentration calibrator. which is used to calculate the albumin concentration of a high-concentration sample.

For each low concentration calibrator: 100 uL of the first reagent R1 was added to a reaction cup; then 6 uL of this low concentration calibrator was further added to the reaction cup; the absorbance S1L was read at a wavelength of 340 nm; then 30 uL of the second reagent R2 was further added to the reaction cup; then the absorbance S2 was read at the wavelength of 340 nm; finally the signal value was calculated: SL = S2-S1L*(100+6)/(100+6+30).

A low-range calibration curve was fitted using the albumin concentration and signal value SL of each low-concentration calibrator, which is used to calculate the albumin concentration of a low-concentration sample.

### 3.3 Protein detection method according to the disclosure

100 uL of the first reagent R1 was added to a reaction cup;
the absorbance S0 was read at the wavelength of 605 nm;
6 uL of a urine sample was further added to the reaction cup;
the absorbance S1H was read at the wavelength of 605 nm, while the absorbance S1L was read at the wavelength of 340;
the signal value was calculated: SH = S1H-SO*100/(100+6), and the albumin concentration value CH corresponding to this signal value was determined by means of the above-mentioned high-range calibration curve;
if the albumin concentration value CH was within a predetermined range, for example above the above-mentioned predetermined threshold value, the albumin measurement result was CH;
if the albumin concentration value CH was not within the predetermined range, for example below the above-mentioned predetermined threshold value, 30 uL of the second reagent R2 was further added to the reaction cup;
the absorbance S2 was read at the wavelength of 340 and the signal value was calculated: SL = S2-S1L*(100+6)/(100+6+30), and the concentration value CL corresponding to this signal value was determined by means of the above-mentioned low-range calibration curve, i.e. the albumin measurement result was CL.

### 3.4 Effect

Multiple urine samples with known albumin concentrations were detected on the Mindray BS-2000 Biochemical Analyzer using the protein analysis reagent according to the disclosure and the protein detection method according to the disclosure, so as to verify the advantages of the technical solution proposed in the examples of the disclosure.

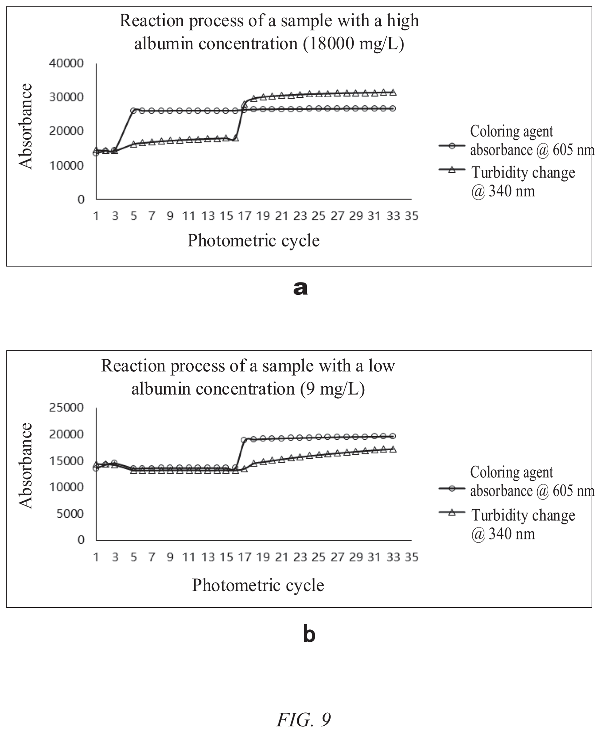

FIG. 9a shows a reaction process during detection of a urine sample with a high albumin concentration (with an albumin concentration of 18000 mg/L). For this urine sample with a high albumin concentration, there is a significant change in absorbance at the wavelength of 605 nm after adding the first reagent R1 in the 5th photometric cycle, which can be used to calculate the albumin concentration. There is an increase in absorbance at the wavelength of 340 nm after adding the second reagent R2 in the 17th photometric cycle, exceeding the detection range of the instrument and failing to provide an accurate result.

FIG. 9b shows a reaction process during detection of a urine sample with a low albumin concentration (with an albumin concentration of 9 mg/L). For the urine sample with a low albumin concentration, there is no significant change in absorbance at the wavelength of 605 nm after adding the first reagent R1 in the 5th photometric cycle, and there is a significant change in absorbance at the wavelength of 340 nm after adding the second reagent R2 in the 17th photometric cycle, with the magnitude of the change being within the detection range, which can be used to calculate the albumin concentration.

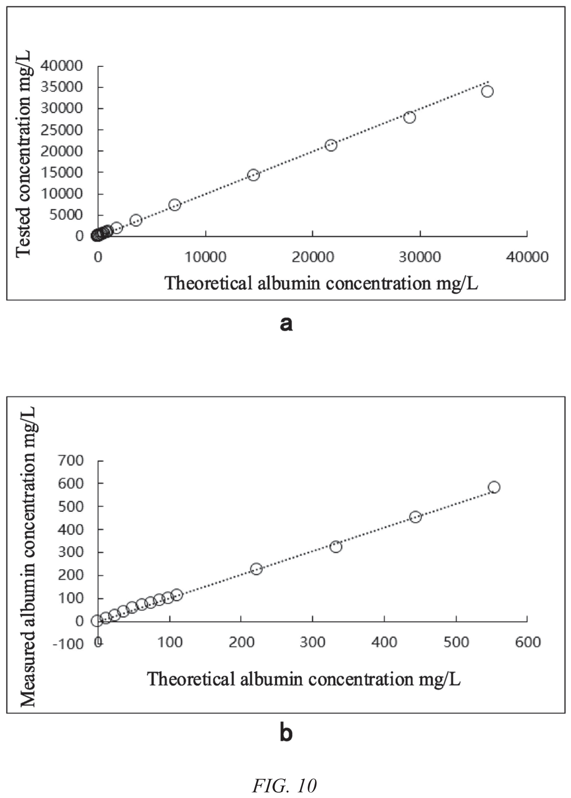

FIG. 10a shows a linear relationship between the known albumin concentrations and the measured albumin concentrations based on the example of the disclosure of all urine samples among these multiple urine samples, and FIG. 10b is a linear relationship between the known albumin concentrations and the measured albumin concentrations based on the example of the disclosure of urine samples with relatively low albumin concentrations.

As can be seen from FIG. 10, the linear detection range for albumin achieved by the example of the disclosure can cover at least 10-40000 mg/L, improving the efficiency of albumin detection and saving the costs incurred by dilution and retesting.

### Example 4 Detection of total protein in urine based on a colorimetric method according to the prior art

### 4.1 Colorimetric reagent

### Pyrogallol red

### Molybdate

### Buffer

4.2 Colorimetric method-based detection method: 100 uL of the colorimetric reagent was added to a reaction cup, the absorbance S0 (absorbance at 570 nm)was read at a primary wavelength of 570 nm, and then 2 uL of a urine sample was further added to the reaction cup, the S1 (absorbance at 570 nm) was read after about 3 minutes at the primary wavelength of 570 nm. The final obtained signal value is S = S1-S0*100/(100+2) and the total protein concentration value corresponding to the obtained signal value was determined by a calibration curve.

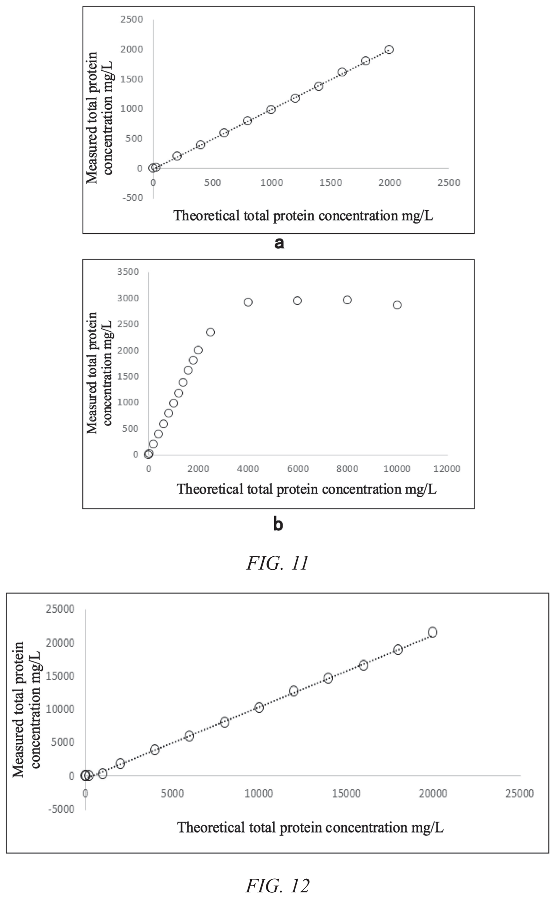

4.3 Multiple urine samples with known total protein concentrations were detected on the Mindray BS-2000 Biochemical Analyzer using the colorimetric reagent and colorimetric method-based detection method described above, so as to obtain measured total protein concentration based on the colorimetric method. FIG. 11 shows a linear relationship between the known total protein concentrations (theoretical total protein concentrations) and the measured total protein concentrations based on the colorimetric method of these multiple urine samples. FIG. 11a shows a linear relationship between the known total protein concentrations and the measured total protein concentrations based on the colorimetric method of the urine samples with relatively low total protein concentrations, while FIG. 11b shows a linear relationship between the known total protein concentrations and the measured total protein concentrations based on the colorimetric method of all urine samples among these multiple urine samples.

As can be seen from FIG. 11a, the colorimetric method has a better linearity when detecting urine samples with total protein concentrations within 2000 mg/L. However, as can be seen in FIG. 11b, when detecting urine samples with relatively high total protein concentrations, there is a lack of reactants and the reaction signal no longer increases with the increase in the analyte.

### Example 5 Detection of total protein in urine based on a turbidimetric method according to the prior art

### 5.1 Turbidimetric reagent

### Sulfosalicylic acid

### Sodium sulfate

5.2 Turbidimetric method-based detection method: 100 uL of the turbidimetric reagent was added to a reaction cup, the absorbance S0 (absorbance at 570 nm) was read at a primary wavelength (570 wavelength is exemplified here, other wavelengths could be used), and then 2 uL of a urine sample was further added to the reaction cup, the S1 (absorbance at 570 nm) was read at the primary wavelength after incubation for about 4 minutes. The final obtained signal value is S = S1-S0*100/(100+2) and the total protein concentration value corresponding to the obtained signal value was determined by a calibration curve.

5.3 Multiple urine samples with known total protein concentrations were detected on the Mindray BS-2000 Biochemical Analyzer using the turbidimetric reagent and turbidimetric method-based detection method described above, so as to obtain measured total protein concentrations based on the turbidimetric method. FIG. 12 shows a linear relationship between the known total protein concentrations (theoretical total protein concentrations) and the measured total protein concentrations based on the turbidimetric method of these multiple urine samples.

As can be seen from FIG. 12, total protein in a urine sample to be tested is precipitated by sulfosalicylic acid and the turbidity is measured, the linear range can reach more than 20000 mg/L. However, in the low total protein concentration range, such as 1000 mg/L, the signal is relatively low and not suitable for detecting urine samples with low total protein concentrations.

### Example 6 Detection of total protein in urine using a combination of a colorimetric method and a precipitation turbidimetric method according to the disclosure

### 6.1 Protein analysis reagent according to the disclosure

### First reagent R1: containing pyrogallol red, molybdate and buffer

### Second reagent R2: containing sulfosalicylic acid and sodium sulfate

### 6.2 Calibration method

A series of calibrators with known total protein concentrations or assigned to determined total protein concentrations were used as samples, the calibrators are classified into high-concentration calibrators (with total protein concentrations above a predetermined threshold value) and low-concentration calibrators (with total protein concentrations not above the predetermined threshold value) by the predetermined threshold value.

For each low concentration calibrator: 100 uL of the first reagent R1 was added to a reaction cup; the absorbance S0 was read at a wavelength of 570 nm; then 2 uL of this low concentration calibrator was further added to the reaction cup; the absorbance S1 was read at the wavelength of 570 nm; finally the signal value was calculated: SL = S1-S0*100/(100+2).

A low-range calibration curve was fitted using the total protein concentration and signal value SL of each low-concentration calibrator, which is used to calculate the total protein concentration of a low-concentration sample.

For each high concentration calibrator: 100 uL of the first reagent R1 was added to a reaction cup, the absorbance S0 was read at the wavelength of 570 nm; then 2 uL of this high concentration calibrator and 100 uL of the second reagent R2 were further added to the reaction cup; then the absorbance S2 was read at the wavelength of 570 nm; finally the signal value was calculated: SH = S2-S0*100/(100+2+100).

A high-range calibration curve was fitted using the total protein concentration and signal value SH of each high-concentration calibrator, which is used to calculate the albumin concentration of a low-concentration sample.

### 6.3 Protein detection method according to the disclosure

100 uL of the first reagent R1 was added to a reaction cup;
the absorbance S0 was read at the wavelength of 570 nm;
2 uL of a urine sample was further added to the reaction cup;
the absorbance S1 was read at the wavelength of 570 nm;
a signal value was calculated: SL = S1-S0*100/(100+2), and the total protein concentration value CL corresponding to this signal value was determined by means of the above-mentioned low-range calibration curve;
100 uL of the second reagent R2 was further added to the reaction cup;
the absorbance S2 was read at the wavelength of 570 and the signal value was calculated: SL = S2-S0*100/(100+2+100), and the concentration value CH corresponding to this signal value was determined by means of the above-mentioned high-range calibration curve.
if the total protein concentration value CL was within a predetermined range, for example below the above-mentioned predetermined threshold value, the total protein measurement result was CH;
if the total protein concentration value CL was not within the predetermined range, for example not below the above-mentioned predetermined threshold value, the total protein measurement result was CH.

### 6.4 Effect

Multiple urine samples with known total protein concentrations were detected on the Mindray BS-2000 Biochemical Analyzer using the protein analysis reagent according to the disclosure and the protein detection method according to the disclosure, so as to verify the advantages of the technical solution proposed in the example of the disclosure.

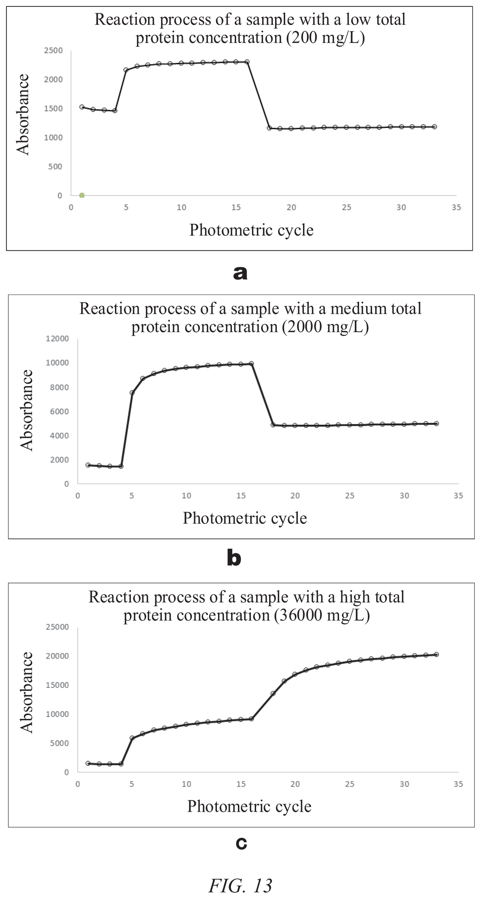

FIG. 13a shows a reaction process during detection of a urine sample with a low total protein concentration (with a total protein concentration of 200 mg/L). For this urine sample with a low total protein concentration, there is sufficient change in absorbance after the first reagent R1 is mixed with this sample, however, after adding the second reagent R2, the absorbance does not change significantly.

FIG. 13b shows a reaction process during detection of a urine sample with a medium total protein concentration (with a total protein concentration of 2000 mg/L). For this urine sample with a medium total protein concentration, there is large change in absorbance after the first reagent R1 is mixed with this sample, however, after adding the second reagent R2, the absorbance does not change significantly.

FIG. 13c shows a reaction process during detection of a urine sample with a high total protein concentration (with a total protein concentration of 36000 mg/L). For this urine sample with a high total protein concentration, although there is a change in absorbance after mixing the first reagent R1 with the sample, the magnitude of the change is close to that of the medium concentration (2000 mg/L), and it could not be distinguished well. However, the absorbance changes significantly after adding the second reagent R2.

FIG. 14a shows a linear relationship between the known total protein concentrations and the measured total protein concentrations based on the example of the disclosure of all urine samples among these multiple urine samples, and FIG. 14b shows a linear relationship between the known albumin concentrations and the measured total protein concentrations based on the example of the disclosure of the urine samples with relatively lower total protein concentrations.

As can be seen from FIG. 14, the linear detection range for total protein achieved by the example of the disclosure can cover at least 20-40000 mg/L, improving the efficiency of total protein detection and saving the costs incurred by dilution and retesting.

The features or combinations thereof mentioned above in the description, accompanying drawings, and claims can be combined with each other arbitrarily or used separately as long as they are meaningful within the scope of the disclosure and do not contradict each other. The advantages and features described for the sample analyzer provided in the embodiments of the disclosure are applicable in a corresponding manner to the protein analysis methods and protein analysis reagent and applications thereof provided in the embodiments of the disclosure, and vice versa.

The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

## Claims

1. A sample analyzer, comprising:
a sample preparation apparatus configured to prepare a test solution to be tested from a sample and a detection reagent;
a detection device configured to detect the test solution to be tested, so as to obtain a detection result for protein in the test solution to be tested;
a controller configured to: control the sample preparation apparatus to prepare a first test solution by using a sample to be tested and a colorimetric reagent, wherein the colorimetric reagent comprises at least a coloring agent, and control the detection device to detect the first test solution by using a colorimetric method, so as to acquire a colorimetric detection result for protein in the sample to be tested;
**characterized in that** the controller is further configured to:
determine, on the basis of the colorimetric detection result, whether to perform a turbidimetric test, if no, then determine a concentration of the protein in the sample to be tested on the basis of the colorimetric detection result, and if yes, then control the sample preparation apparatus to prepare a second test solution by using the sample to be tested and a turbidimetric reagent, and control the detection device to detect the second test solution by using a turbidimetric method, so as to acquire a turbidimetric detection result for the protein in the sample to be tested, and determine a concentration of the protein in the sample to be tested on the basis of the turbidimetric detection result and optionally the colorimetric detection result; or,
control the sample preparation apparatus to prepare a second test solution by using the sample to be tested and a turbidimetric reagent, and control the detection device to detect the second test solution by using a turbidimetric method, so as to acquire a turbidimetric detection result for the protein in the sample to be tested, and select, on the basis of the colorimetric detection result, at least one of the colorimetric detection result and the turbidimetric detection result to determine a concentration of the protein in the sample to be tested.

2. The sample analyzer of claim 1, **characterized in that** the turbidimetric reagent comprises at least an antibody reagent;
the controller further configured to determine, on the basis of the colorimetric detection result, whether to perform a turbidimetric test, comprises: the controller further configured to:
determine that the turbidimetric test is not required to be performed, if the colorimetric detection result is in a first predetermined range, for example greater than a predetermined threshold value; and
determine that the turbidimetric test is required to be performed, if the colorimetric detection result is in a second predetermined range, for example not greater than the predetermined threshold value.

3. The sample analyzer of claim 1, **characterized in that** the turbidimetric reagent comprises at least an antibody reagent;
the controller further configured to select, on the basis of the colorimetric detection result, at least one of the colorimetric detection result and the turbidimetric detection result to determine a concentration of the protein in the sample to be tested, comprises: the controller further configured to:
calculate the concentration of the protein in the sample to be tested only on the basis of the colorimetric detection result, if the colorimetric detection result is in a first predetermined range, for example greater than a predetermined threshold value;
calculate the concentration of the protein in the sample to be tested only on the basis of the turbidimetric detection result, if the colorimetric detection result is in a second predetermined range, for example not greater than the predetermined threshold value.

4. The sample analyzer of claim 1, **characterized in that** the turbidimetric reagent comprises at least an antibody reagent; and
the controller further configured to select, on the basis of the colorimetric detection result, at least one of the colorimetric detection result and the turbidimetric detection result to determine a concentration of the protein in the sample to be tested, comprises: the controller further configured to:
calculate the concentration of the protein in the sample to be tested only on the basis of the colorimetric detection result, if the colorimetric detection result is in a first predetermined range, for example greater than a predetermined threshold value by a first difference value;
calculate the concentration of the protein in the sample to be tested only on the basis of the turbidimetric detection result, if the colorimetric detection result is in a second predetermined range, for example less than the predetermined threshold value by a second difference value;
calculate the concentration of the protein in the sample to be tested on the basis of the turbidimetric detection result and the colorimetric detection result, if the colorimetric detection result is in a third predetermined range, for example, an absolute difference between the colorimetric detection result and the predetermined threshold value is not greater than the first difference value and the second difference value.

5. The sample analyzer of claim 1, **characterized in that** the turbidimetric reagent comprises at least a protein precipitation reagent;
the controller further configured to determine, on the basis of the colorimetric detection result, whether to perform a turbidimetric test, comprises: the controller further configured to:
determine that the turbidimetric test is not required to be performed, if the colorimetric detection result is in a first predetermined range, for example less than a predetermined threshold value; and
determine that the turbidimetric test is required to be performed, if the colorimetric detection result is in a second predetermined range, for example not less than the predetermined threshold value.

6. The sample analyzer of claim 1, **characterized in that** the turbidimetric reagent comprises at least a protein precipitation reagent;
the controller further configured to select, on the basis of the colorimetric detection result, at least one of the colorimetric detection result and the turbidimetric detection result to determine a concentration of the protein in the sample to be tested, comprises: the controller further configured to:
calculate the concentration of the protein in the sample to be tested only on the basis of the colorimetric detection result, if the colorimetric detection result is in a first predetermined range, for example less than a predetermined threshold value;
calculate the concentration of the protein in the sample to be tested only on the basis of the turbidimetric detection result, if the colorimetric detection result is in a second predetermined range, for example not less than the predetermined threshold value.

7. The sample analyzer of claim 1, **characterized in that** the turbidimetric reagent comprises at least a protein precipitation reagent; and
the controller further configured to select, on the basis of the colorimetric detection result, at least one of the colorimetric detection result and the turbidimetric detection result to determine a concentration of the protein in the sample to be tested, comprises: the controller further configured:
calculate the concentration of the protein in the sample to be tested only on the basis of the colorimetric detection result, if the colorimetric detection result is in a first predetermined range, for example less than a predetermined threshold value by a first difference value;
calculate the concentration of the protein in the sample to be tested only on the basis of the turbidimetric detection result, if the colorimetric detection result is in a second predetermined range, for example greater than the predetermined threshold value by a second difference value;
calculate the concentration of the protein in the sample to be tested on the basis of the turbidimetric detection result and the colorimetric detection result, if the colorimetric detection result is in a third predetermined range, for example, an absolute difference between the colorimetric detection result and the predetermined threshold value is not greater than the first difference value and the second difference value.

8. The sample analyzer of any one of claims 1 to 7, **characterized in that**
the controller configured to control the sample preparation apparatus to prepare a first test solution by using a sample to be tested and a colorimetric reagent, comprises: the controller configured to control the sample preparation apparatus to mix at least a portion of the sample to be tested with the colorimetric reagent to prepare the first test solution; and
the controller further configured to control the sample preparation apparatus to prepare a second test solution by using the sample to be tested and a turbidimetric reagent, comprises: the controller further configured to control the sample preparation apparatus to mix at least a portion of the first test solution with the turbidimetric reagent to prepare the second test solution.

9. The sample analyzer of any one of claims 1 to 7, **characterized in that**
the controller configured to control the sample preparation apparatus to prepare a first test solution by using a sample to be tested and a colorimetric reagent, comprises: the controller configured to control the sample preparation apparatus to mix at least a portion of the sample to be tested with the colorimetric reagent to prepare the first test solution; and
the controller further configured to control the sample preparation apparatus to prepare a second test solution by using the sample to be tested and a turbidimetric reagent, comprises: the controller further configured to control the sample preparation apparatus to mix at least another portion of the sample to be tested with the turbidimetric reagent to prepare the second test solution.

10. The sample analyzer of any one of claims 1 to 9, **characterized in that**
the protein is albumin or total protein, preferably the protein is albumin; and/or the sample to be tested is urine or serum or cerebrospinal fluid, preferably the sample to be tested is urine;
preferably, the sample to be tested is urine and the protein is albumin or total protein.

11. A sample analyzer, comprising:
a sample preparation apparatus configured to prepare a test solution to be tested from a sample;
a detection device configured to detect the test solution to be tested, so as to obtain a detection result for protein in the test solution to be tested;
a controller configured to: on the basis of a first detection method, control the sample preparation apparatus to prepare a first test solution by using a sample to be tested and a first detection reagent, and control the detection device to detect the first test solution, so as to acquire a first detection result for protein in the sample to be tested;
**characterized in that** the controller is further configured to:
determine, on the basis of the first detection result, whether to perform a test on the basis of a second detection method, if no, then determine a concentration of the protein in the sample to be tested on the basis of the first detection result, if yes, then on the basis of the second detection method, control the sample preparation apparatus to prepare a second test solution by using the sample to be tested, and control the detection device to detect the second test solution, so as to acquire a second detection result for the protein in the sample to be tested, and determine a concentration of the protein in the sample to be tested on the basis of the second detection result and the first detection result; or,
on the basis of the second detection method, control the sample preparation apparatus to prepare a second test solution by using the sample to be tested, and control the detection device to detect the second test solution, so as to acquire a second detection result for the protein in the sample to be tested, and select, on the basis of the first detection result, at least one of the first detection result and the second detection result to determine a concentration of the protein in the sample to be tested.

12. A sample analyzer, comprising:
a sample preparation apparatus configured to prepare a test solution to be tested from a sample;
a detection device configured to detect the test solution to be tested, so as to obtain a detection result for protein in the test solution to be tested;
a controller configured to: on the basis of a first detection method, control the sample preparation apparatus to prepare a first test solution by using a sample to be tested and a first detection reagent, and control the detection device to detect the first test solution, so as to acquire a first detection result for protein in the sample to be tested;
**characterized in that** the controller is further configured to:
determine, on the basis of the first detection result, whether to perform a test on the basis of a second detection method, if no, then determine a concentration of the protein in the sample to be tested on the basis of the first detection result, if yes, then on the basis of the second detection method, control the sample preparation apparatus to prepare a second test solution by using the sample to be tested and a second detection reagent, and control the detection device to detect the second test solution, so as to acquire a second detection result for the protein in the sample to be tested, and determine a concentration of protein in the sample to be tested on the basis of the second detection result and optionally the first detection result; or,
on the basis of the second detection method, control the sample preparation apparatus to prepare a second test solution by using the sample to be tested and a second detection reagent, and control the detection device to detect the second test solution, so as to acquire a second detection result for the protein in the sample to be tested, and select, on the basis of the first detection result, at least one of the first detection result and the second detection result to determine a concentration of the protein in the sample to be tested.

13. The sample analyzer of claim 11 or 12, **characterized in that** a protein concentration detection upper limit of the first detection method is greater than a protein concentration detection upper limit of the second detection method; the controller further configured to determine, on the basis of the first detection result, whether to perform a test on the basis of a second detection method, comprises: controller further configured to: determine that the test on the basis of the second detection method is not required to be performed, if the first detection result is greater than a predetermined threshold value; and determine that the test on the basis of the second detection method is required to be performed, if the first detection result is not greater than the predetermined threshold value; or,
**characterized in that** a protein concentration detection upper limit of the first detection method is less than a protein concentration detection upper limit of the second detection method; the controller further configured to determine, on the basis of the first detection result, whether to perform a test on the basis of a second detection method, comprises: controller further configured to: determine that the test on the basis of the second detection method is not required to be performed, if the first detection result is less than a predetermined threshold value; and determine that the test on the basis of the second detection method is required to be performed, if the first detection result is not less than the predetermined threshold value.

14. The sample analyzer of any one of claims 11 to 13, **characterized in that** a calibration curve corresponding to the first detection method and a calibration curve corresponding to the second detection method are different;
the controller is further configured to:
obtain the first detection result from a first calibration curve and first optical signals obtained by detecting the first test solution;
obtain the second detection result from a second calibration curve and second optical signals obtained by detecting the second test solution;
wherein the first calibration curve is different from the second calibration curve, preferably a linear range of the first calibration curve is different from that of the second calibration curve, and the linear range of the first calibration curve and the linear range of the second calibration curve partially overlap with each other.

15. The sample analyzer of any one of claims 11 to 13, **characterized in that** the first detection method and the second detection method are based on different methodologies; or,
the first detection reagent used in the first detection method and the second detection reagent used in the second detection method belong to different categories; or,
the first detection reagent used in the first detection method and the second detection reagent used in the second detection method belong to a same category, but the first detection reagent used in the first detection method and the second detection reagent used in the second detection method differ in formulation, especially in concentration; or,
a detector used for optical signal measurement of the first test solution in the first detection method and a detector used for optical signal measurement of the second test solution in the second detection method belong to different categories or have different linear detection ranges; or,
a detection wavelength used for optical signal measurement of the first test solution in the first detection method is different from a detection wavelength used for optical signal measurement of the second test solution in the second detection method.

16. A protein analysis method for a sample analyzer, wherein the sample analyzer comprises a sample preparation apparatus, a detection device, and a controller, and the protein analysis method comprises: controlling, by the controller, the sample preparation apparatus to prepare a first test solution by using a sample to be tested and a colorimetric reagent, wherein the colorimetric reagent comprises at least a coloring agent; and controlling, by the controller, the detection device to detect the first test solution by using a colorimetric method, so as to acquire a colorimetric detection result for protein in the sample to be tested;
**characterized in that** the protein analysis method further comprises performing the following steps by the controller:
determining, on the basis of the colorimetric detection result, whether to perform a turbidimetric test, if no, then determining a concentration of the protein in the sample to be tested on the basis of the colorimetric detection result, and if yes, then controlling the sample preparation apparatus to prepare a second test solution by using the sample to be tested and a turbidimetric reagent, and controlling the detection device to detect the second test solution by using a turbidimetric method, so as to acquire a turbidimetric detection result for the protein in the sample to be tested, and determining a concentration of the protein in the sample to be tested on the basis of the turbidimetric detection result and optionally the colorimetric detection result; or,
controlling the sample preparation apparatus to prepare a second test solution by using the sample to be tested and a turbidimetric reagent, and controlling the detection device to detect the second test solution by using a turbidimetric method, so as to acquire a turbidimetric detection result for the protein in the sample to be tested, selecting, on the basis of the colorimetric detection result, at least one of the colorimetric detection result and the turbidimetric detection result to determine a concentration of the protein in the sample to be tested.

17. A protein analysis method for a sample analyzer, wherein the sample analyzer comprises a sample preparation apparatus, a detection device, and a controller, and the protein analysis method comprises: controlling, by the controller, the sample preparation apparatus to prepare a first test solution by using a sample to be tested and a turbidimetric reagent; and controlling by the controller, the detection device to detect the first test solution by using a turbidimetric method, so as to acquire a turbidimetric detection result for protein in the sample to be tested;
**characterized in that** the protein analysis method further comprises performing the following steps by the controller:
determining, on the basis of the turbidimetric detection result, whether to perform a colorimetric test, if no, then determining a concentration of the protein in the sample to be tested on the basis of the colorimetric detection result, and if yes, then controlling the sample preparation apparatus to prepare a second test solution by using the sample to be tested and a colorimetric reagent, and controlling the detection device to detect the second test solution by using a colorimetric method, so as to acquire a colorimetric detection result for the protein in the sample to be tested, and determining a concentration of the protein in the sample to be tested on the basis of the colorimetric detection result and optionally the turbidimetric detection result, wherein the colorimetric reagent comprises at least a coloring agent; or,
controlling the sample preparation apparatus to prepare a second test solution by using the sample to be tested and a colorimetric reagent, controlling the detection device to detect the second test solution by using a colorimetric method, so as to acquire a colorimetric detection result for the protein in the sample to be tested, wherein the colorimetric reagent comprises at least a coloring agent, and selecting, on the basis of the turbidimetric detection result, at least one of the colorimetric detection result and the turbidimetric detection result to determine a concentration of the protein in the sample to be tested.

18. A protein analysis reagent, **characterized in that** the protein analysis reagent comprises:
a colorimetric reagent comprising a coloring agent; and
a turbidimetric reagent comprising an antibody reagent or a protein precipitation reagent.

19. The protein analysis reagent of claim 18, **characterized in that** the colorimetric reagent further comprises a turbidity-enhancing reagent comprising a hydrophilic polymer; the turbidimetric reagent comprises an antibody reagent.

20. The protein analysis reagent of claim 18, **characterized in that** the turbidimetric reagent comprises an antibody reagent, wherein the antibody reagent comprises a solid-phase carrier with an antibody attached to a surface thereof, wherein the solid-phase carrier is preferably latex microspheres.

21. The protein analysis reagent of claim 19, **characterized in that** the coloring agent and the turbidity-enhancing reagent are mixed and packaged as a first reagent, and the turbidimetric reagent is packaged as a second reagent; or the coloring agent, the turbidity-enhancing reagent and the turbidimetric reagent are separately and independently packaged.
